# EUROPEAN PATENT APPLICATION

(11) **EP 0 918 056 A1**
(43) Date of publication of application: **26.05.1999**
(21) Application number: 98121707.8
(22) Date of filing: 13.11.1998
(51) Int. Cl.: C07D 411/06, C07D 409/06, C07D 495/06, C07D 498/04, C07D 327/04, C07D 327/06, C07D 327/02, C07D 335/06, A01N 43/32, A01N 43/80

(54) **Herbicides**

(30) Priority: 20.11.1997 GB 9724586
(71) Applicant: RHONE-POULENC AGRICULTURE LTD., Ongar, Essex CM5 0HW (GB)
(72) Inventor: Cramp, Susan Mary, Ongar, Essex CM5 OHW (GB); Geach, Neil Jonathan, Ongar, Essex CM5 OHW (GB)
(74) Representative: Brachotte, Charles Yves

(57) **Abstract**

The invention relates to 4-aroylisoxazole derivatives of formula (Ia) or to 2-cyano-1,3-dione derivatives of formula (Ib): 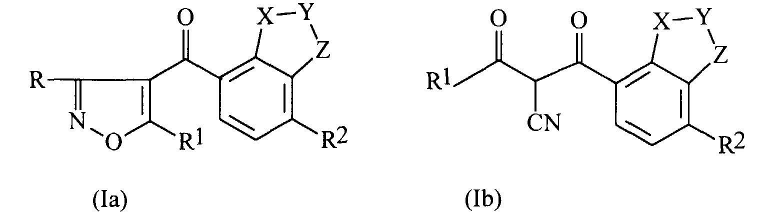 wherein R, R¹, R², X, Y and Z are as defined in the description, and to their use as herbicides.

## Description

This invention relates to novel 4-aroylisoxazole derivatives, 2-cyano-1,3-dione derivatives, compositions containing them, processes for their preparation, intermediates in their preparation and their use as herbicides.

Herbicidal 4-aroylisoxazoles are described in a number of patent publications for example European Patent Publication Numbers 0418175, 0487357, 0527036, 0527037, 0560482 , 0560483 and 0636622, as well as International Patent Publication Numbers WO 96/25413, 97/19076 and 97/19087. Herbicidal 2-cyano-1,3-diones are described in European Patent Publication Numbers 0213892, 0496630 and 0496631, and International Patent Publication Numbers WO 95/25099, 96/05198, 97/13765, 97/19071 and 97/19087.

The present invention provides 4-aroylisoxazole derivatives of formula (Ia) and 2-cyano-1,3-dione derivatives of formula (Ib): 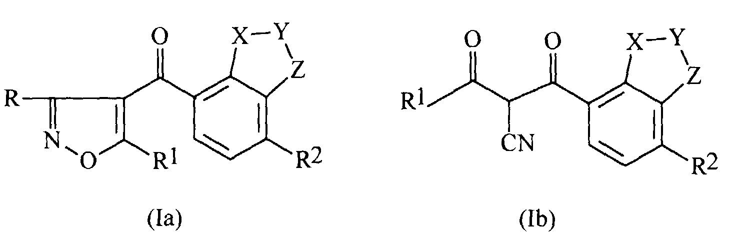 wherein:
R represents hydrogen or -CO₂R³;
R¹ represents:-
   cyclopropyl or 1-methylcyclopropyl;
R² represents:-
   halogen, lower alkyl, lower haloalkyl, lower alkoxy, lower haloalkoxy, -S(O)ₘR⁴ or SF₅;
m represents zero, one or two;
X, Y and Z together with the two attached carbon atoms form a fused 5- to 7- membered heterocyclic ring which is aromatic or partially or fully saturated containing from one to three heteroatoms which may be the same or different selected from nitrogen, oxygen and sulphur with at least one atom being a sulphur atom (the sulphur atoms being optionally in the SO or SO₂ oxidation states), and the ring carbon atoms being optionally substituted by one or two groups selected from halogen, lower alkyl, lower haloalkyl, lower alkoxy and lower haloalkoxy, one of the ring carbon atoms optionally forming part of a carbonyl group or an oxime or lower alkoxyimine derivative thereof; and optionally two of the adjacent carbon atoms which when present form part of the X-Y-Z group in the heterocyclic ring may themselves form part of a second fused pyrazole or isoxazole ring optionally substituted by lower alkyl, lower haloalkyl, halogen or -CO₂R⁵;
R³ represents lower alkyl or lower haloalkyl;
R⁴ represents lower alkyl, lower haloalkyl, lower alkenyl, lower haloalkenyl, lower alkynyl, lower haloalkynyl or cycloalkyl containing from three to six carbon atoms;
R⁵ represents lower alkyl;
and agriculturally acceptable salts and metal complexes thereof, which possess valuable herbicidal properties.

Compounds of formula (Ib) may exist in enolic tautomeric forms that may give rise to geometric isomers around the enolic double bond. Furthermore in certain cases the substituents R, and R¹ to R⁵ may contribute to optical isomerism and/or stereoisomerism. All such forms are embraced by the present invention.

In the description unless otherwise specified the following terms are generally defined thus:-
'lower alkyl' means a straight- or branched- chain alkyl group having one to six carbon atoms;
'lower haloalkyl' means a straight- or branched- chain alkyl group having one to six carbon atoms, substituted by one or more halogens;
'lower alkoxy' means a straight- or branched- chain alkoxy group having one to six carbon atoms;
'lower haloalkoxy' means a straight- or branched- chain alkoxy group having one to six carbon atoms, substituted by one or more halogens;
'lower alkoxyimine' means an imino group substituted by a straight- or branched- chain alkoxy group having one to six carbon atoms;
'lower alkenyl' means a straight- or branched- chain alkenyl group having two to six (preferably three to six) carbon atoms;
'lower haloalkenyl' means a straight- or branched- chain alkenyl group having two to six (preferably three to six) carbon atoms, substituted by one or more halogens;
'lower alkynyl' means a straight- or branched- chain alkynyl group having two to six (preferably three to six) carbon atoms;
'lower haloalkynyl' means a straight- or branched- chain alkynyl group having two to six (preferably three to six) carbon atoms, substituted by one or more halogens;
'halogen' means a fluorine, chlorine, bromine or iodine atom.

In the description that follows, reference to compounds of formula (I) means reference to a compound of formula (Ia) or (Ib).

By the term "agriculturally acceptable salts" is meant salts the cations or anions of which are known and accepted in the art for the formation of salts for agricultural or horticultural use. Preferably the salts are water-soluble. Suitable salts with bases include alkali metal (eg. sodium and potassium), alkaline earth metal (eg. calcium and magnesium), ammonium and amine (eg. diethanolamine, triethanolamine, octylamine, morpholine and dioctylmethylamine) salts. Suitable acid addition salts, formed by compounds of formula (I) containing a basic nitrogen atom in the heterocyclic ring, include salts with inorganic acids, for example hydrochlorides, sulphates, phosphates and nitrates and salts with organic acids, for example acetic acid.

By the term "metal complexes" is meant compounds in which one or both of the oxygen atoms of the 1,3-dione of formula (Ib) act as chelating agents to a metal cation. Examples of such cations include zinc, manganese, cupric, cuprous, ferric, ferrous, titanium and aluminium.

The compounds of the invention, in certain aspects of their properties, for example their control of weeds found in maize and soybean such as Abutilon theophrasti and Amaranthus retroflexus (especially post-emergence) show advantageous properties over known compounds.

Compounds of formula (Ia) above are preferred.

Preferably R represents hydrogen.

Preferably R¹ represents cyclopropyl.

Preferably R² is selected from fluorine, chlorine, bromine, methyl, methoxy and -S(O)ₘMe, (compounds in which R² represents bromine are especially preferred).

Preferably the -X-Y-Z- group together with the phenyl ring to which it is fused forms a ring system which is selected from thiochroman, 2H-thiochromene, 4H-thiochromene, isothiochroman, isothiochromene, 1,3-benzodithiole, 1,3-benzoxathiole, 1,4-benzodithiin, 1,4-benzodithian, 1,4-benzoxathiin, 1,4-benzoxathian, 1,3-benzoxathian, 3,1-benzoxathian, 1,3-benzodithian, benzo[b]thiophene, 2,3-dihydro-benzo[b]thiophene, 3-oxo-2,3-dihydro-benz[d]isothiazole, 1,5-benzoxathiepan, benzothiopyrano[4,3-c]pyrazole, benzothiopyrano[4,3-c]isoxazole and benzothiopyrano[3,4-d]isoxazole (the 1,4-benzoxathian ring system is especially preferred).

A preferred class of compounds are those of formula (Ia) wherein:-
R represents hydrogen or -CO₂R³;
R¹ represents cyclopropyl;
R² represents:-
   halogen; a straight- or branched- chain alkyl group containing up to four carbon atoms optionally substituted by one or more halogen atoms; -S(O)ₘR⁴ or a straight- or branched- chain alkoxy group containing from one to four carbon atoms;
R³ and R⁴ each represent a straight- or branched- chain alkyl group containing from one to four carbon atoms;
X represents S(O)ₙ, O or C(=O);
Y represents (CH₂)ₚ optionally substituted by lower alkyl;
Z represents S(O)_{q}, O, C(=O), C(=NOH), C(=NOCH₃) or CH(OCH₃);
provided that at least one of the groups S(O)ₙ or S(O)_{q} is present as the group X or Z respectively;
n and q independently represent zero, one or two; and
p represents one, two or three; or the -X-Y-Z- group together with the phenyl ring to which it is fused forms a ring system which is selected from benzothiopyrano[4,3-c]pyrazole, benzothiopyrano[4,3-c]isoxazole or benzothiopyrano[3,4-d]isoxazole optionally substituted in the pyrazole or isoxazole ring by lower alkyl or lower alkoxycarbonyl.

A further preferred class of compounds are those of formula (Ia) wherein:-
R represents hydrogen or -CO₂R³;
R¹ represents cyclopropyl;
R² represents:-
   halogen, optionally halogenated methyl, -S(O)ₘCH₃ or OCH₃;
R³ represents methyl or ethyl;
Y represents (CH₂)ₚ optionally substituted by lower alkyl;
p represents one, two or three;
X represents S(O)ₙ and Z represents O, C(=O), C(=NOH), C(=NOCH₃) or CH(OCH₃); or
X represents O or C(=O) and Z represents S(O)_{q}; or the -X-Y-Z-group together with the phenyl ring to which it is fused forms a ring system which is selected from benzothiopyrano[4,3-c]pyrazole, benzothiopyrano[4,3-c]isoxazole or benzothiopyrano[3,4-d]isoxazole optionally substituted in the pyrazole or isoxazole ring by lower alkyl or lower alkoxycarbonyl.

A further preferred class of compounds are those of formula (Ia) wherein:-
R represents hydrogen or -CO₂R³;
R¹ represents cyclopropyl;
R² represents:-
   halogen, optionally halogenated methyl, -S(O)ₘCH₃ or OCH₃;
R³ represents methyl or ethyl;
Y represents (CH₂)ₚ optionally substituted by lower alkyl;
p represents one, two or three; and
X represents S(O)ₙ and Z represents O; or X represents O and Z represents S(O)_{q}.

A most preferred class of compounds are those of formula (Ia) wherein:-
R represents hydrogen;
R¹ represents cyclopropyl;
R² represents chlorine or bromine;
Y represents (CH₂)₂; and
X represents S(O)ₙ and Z represents O; or X represents O and Z represents S(O)_{q}.

Particularly important compounds of the invention include:
4-(5-bromo-1,4-benzoxathian-8-oyl)-5-cyclopropylisoxazole (Compound 1)
4-(5-bromo-1,4-benzoxathian-8-oyl)-5-cyclopropylisoxazole S,S-dioxide (Compound 2)
4-(5-bromo-1,4-benzoxathian-8-oyl)-5-cyclopropylisoxazole S-oxide (Compound 3)
5-cyclopropyl-4-(5-fluoro-1,4-benzoxathian-8-oyl)isoxazole (Compound 4)
5-cyclopropyl-4-(8-methyl-1,4-benzoxathian-5-oyl)isoxazole S-oxide (Compound 5)
5-cyclopropyl-4-(8-bromo-2-methyl-1,4-benzoxathian-5-oyl)isoxazole (Compound 6)
4-(8-chloro-1,4-benzoxathian-5-oyl)-5-cyclopropyl-isoxazole (Compound 7)
4-(8-chloro-1,4-benzoxathian-5-oyl)-5-cyclopropylisoxazole S-oxide (Compound 8)
4-(8-methoxy-1,4-benzoxathian-5-oyl)-5-cyclopropylisoxazole S,S-dioxide (Compound 9)
4-(8-methoxy-1,4-benzoxathian-5-oyl)-5-cyclopropylisoxazole S-oxide (Compound 10)
5-cyclopropyl-4-(8-methoxy-1,4-benzoxathian-5-oyl)isoxazole (Compound 11)
4-(7-bromo-1,3-benzoxathiolan-4-oyl)-5-cyclopropyl-isoxazole (Compound 12)
5-cyclopropyl-4-(8-methyl-1,4-benzoxathian-5-oyl)isoxazole (Compound 13)
4-(8-bromo-3-methyl-1,4-benzoxathian-5-oyl)-5-cyclopropylisoxazole (Compound 14)
5-cyclopropyl-4-(9-fluoro-1,5-benzoxathiepan-6-oyl)isoxazole S,S-dioxide (Compound 15)
5-cyclopropyl-4-(9-fluoro-1,5-benzoxathiepan-6-oyl)isoxazole S-oxide (Compound 16)
5-cyclopropyl-4-(9-fluoro-1,5-benzoxathiepan-6-oyl)isoxazole (Compound 17)
4-(5-chloro-1,4-benzoxathian-8-oyl)-5-cyclopropylisoxazole S,S-dioxide (Compound 18)
4-(5-chloro-1,4-benzoxathian-8-oyl)-5-cyclopropylisoxazole S-oxide (Compound 19)
4-(5-chloro-1,4-benzoxathian-8-oyl)-5-cyclopropylisoxazole (Compound 20)
5-cyclopropyl-4-(5-methylsulphonyl-1,4-benzoxathian-8-oyl)isoxazole S,S-dioxide (Compound 21)
5-cyclopropyl-4-(5-methylthio-1,4-benzoxathian-8-oyl)isoxazole S-oxide (Compound 22)
5-cyclopropyl-4-(5-methylthio-1,4-benzoxathian-8-oyl)isoxazole (Compound 23)
ethyl 5-cyclopropyl-4-(8-fluoro-1,4-benzoxathian-5-oyl)isoxazole-3-carboxylate S,S-dioxide (Compound 24)
ethyl 5-cyclopropyl-4-(8-fluoro-1,4-benzoxathian-5-oyl)isoxazole-3-carboxylate S-oxide (Compound 25)
ethyl 5-cyclopropyl-4-(8-fluoro-1,4-benzoxathian-5-oyl)isoxazole-3-carboxylate (Compound 26)
ethyl 5-cyclopropyl-4-(8-methylthio-1,4-benzoxathian-5-oyl)isoxazole-3-carboxylate (Compound 27)
ethyl 5-cyclopropyl-4-(8-methylsulphonyl-1,4-benzoxathian-5-oyl)isoxazole-3-carboxylate S,S-dioxide (Compound 28)
5-cyclopropyl-4-(8-fluoro-1,4-benzoxathian-5-oyl)isoxazole S,S-dioxide (Compound 29)
5-cyclopropyl-4-(8-fluoro-1,4-benzoxathian-5-oyl)isoxazole S-oxide (Compound 30)
5-cyclopropyl-4-(8-fluoro-1,4-benzoxathian-5-oyl)isoxazole (Compound 31)
5-cyclopropyl-4-(8-methylthio-1,4-benzoxathian-5-oyl)isoxazole (Compound 32)
5-cyclopropyl-4-(8-methylsulphonyl-1,4-benzoxathian-5-oyl)isoxazole S,S-dioxide (Compound 33)
4-(8-bromo-1,4-benzoxathian-5-oyl)-5-cyclopropylisoxazole S,S-dioxide (Compound 34)
4-(8-bromo-1,4-benzoxathian-5-oyl)-5-cyclopropylisoxazole S-oxide (Compound 35)
4-(8-bromo-1,4-benzoxathian-5-oyl)-5-cyclopropylisoxazole (Compound number 36)
1-(8-bromo-1,4-benzoxathian-5-yl)-2-cyano-3-cyclopropyl-propan-1,3-dione S-oxide (Compound number 37) and
5-cyclopropyl-4-(8-methyl-1,4-benzoxathian-5-oyl)isoxazole S,S-dioxide (Compound 38).

The following compounds of formula (Ia) in which R represents hydrogen and R¹ represents cyclopropyl shown in Table 1; and of formula (Id)-(Ik) (shown in Tables 2-9), also form part of the present invention. Note that in the Tables that follow, Me means methyl and Et means ethyl. Where subscripts do not appear in the Tables it will be understood that in appropriate cases they are present (e.g. CH2 means CH₂ etc.).

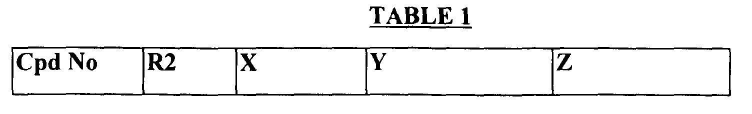

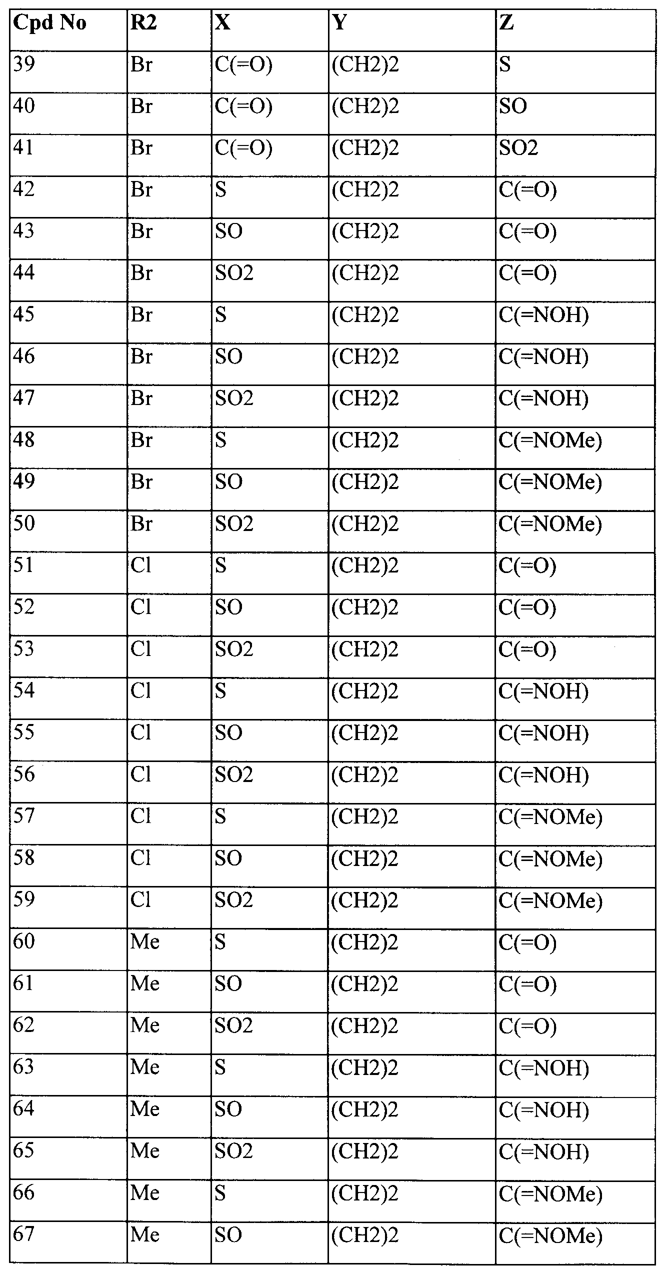

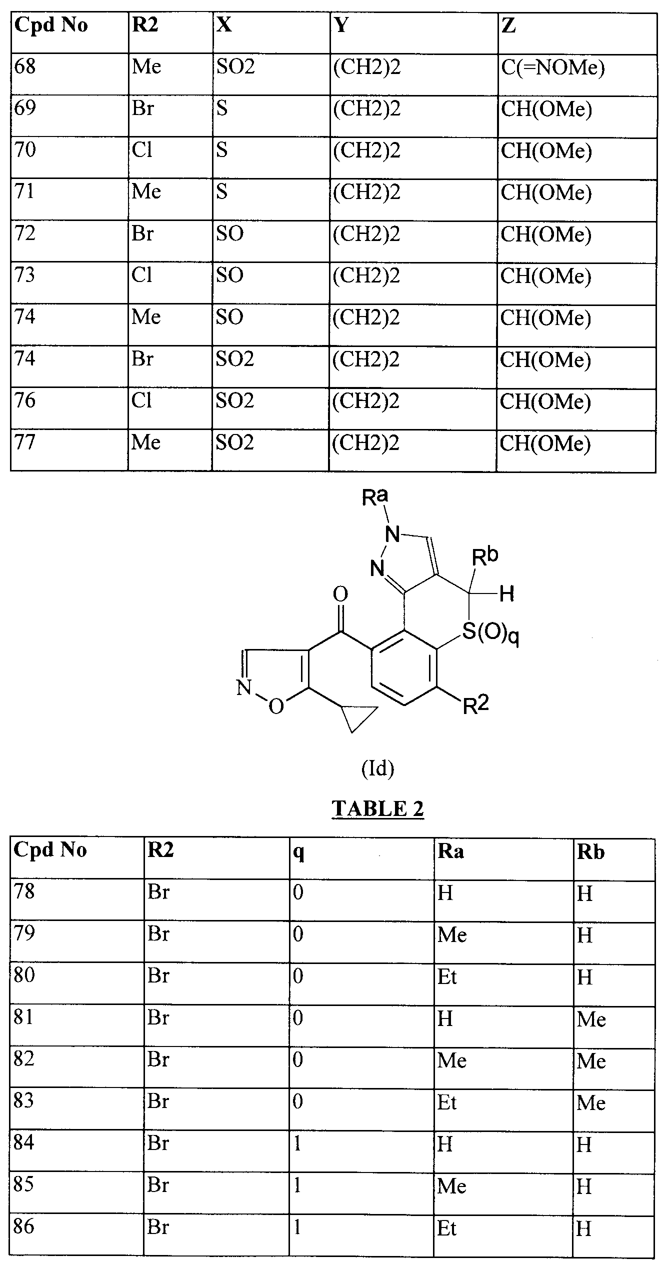

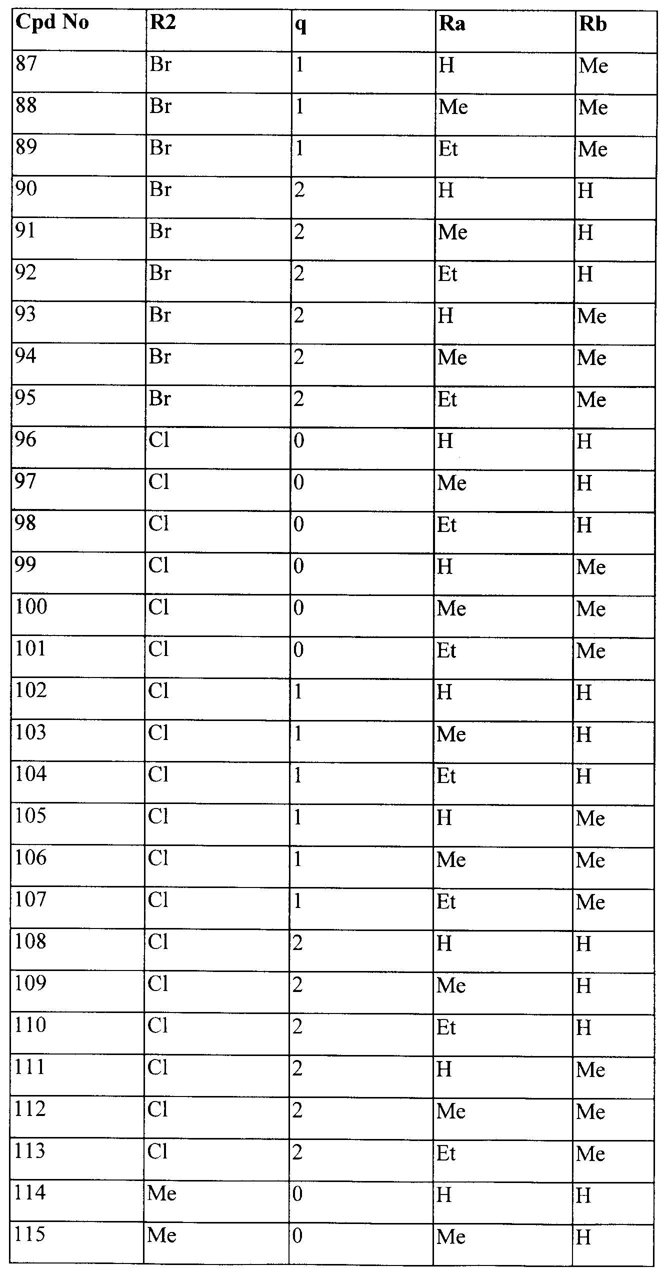

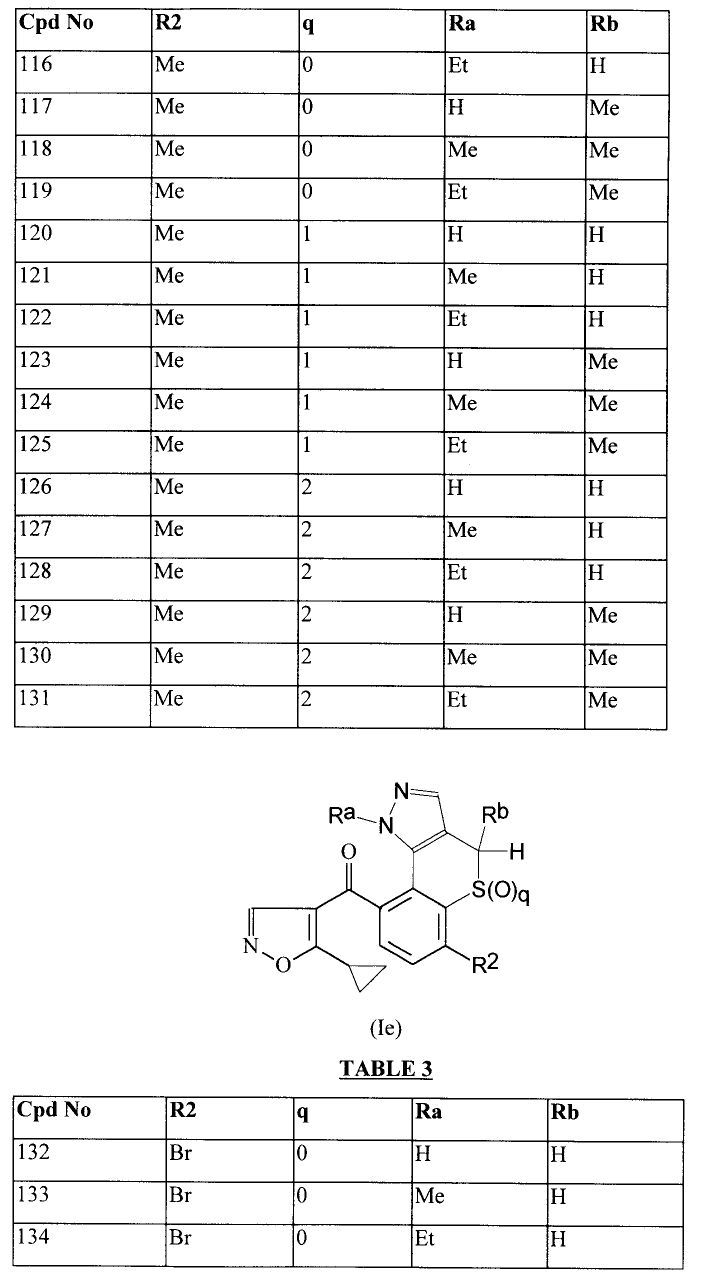

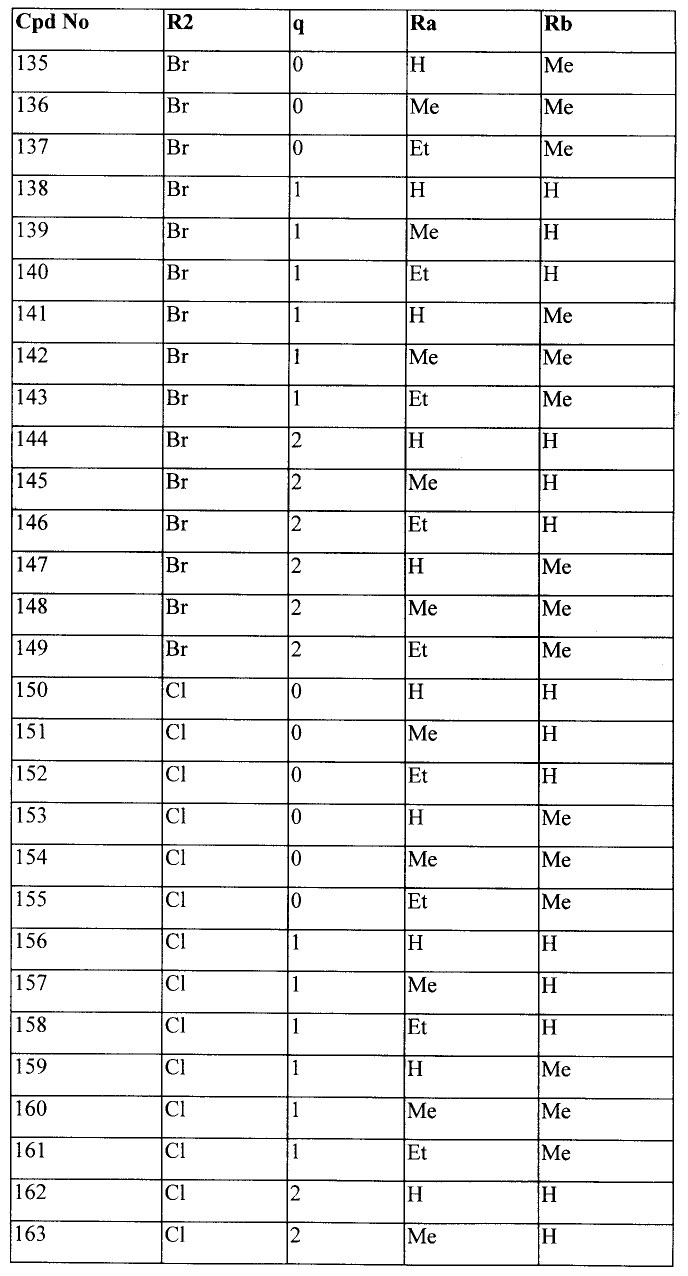

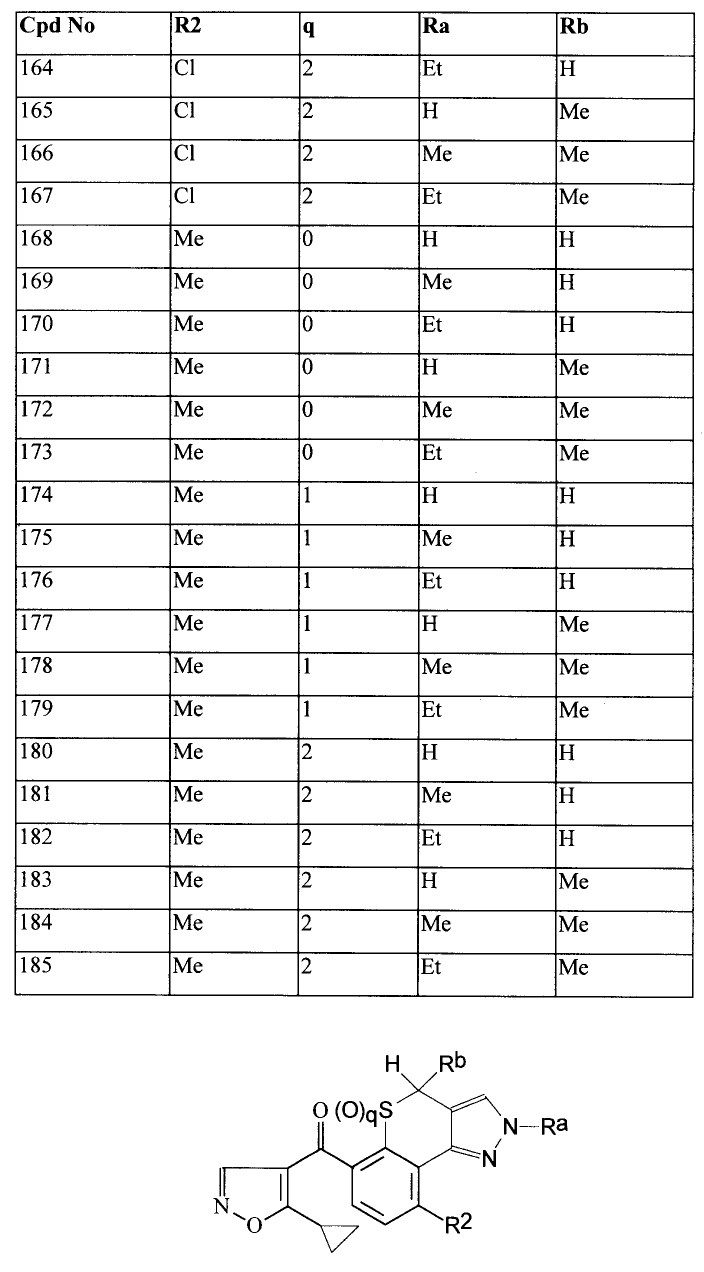

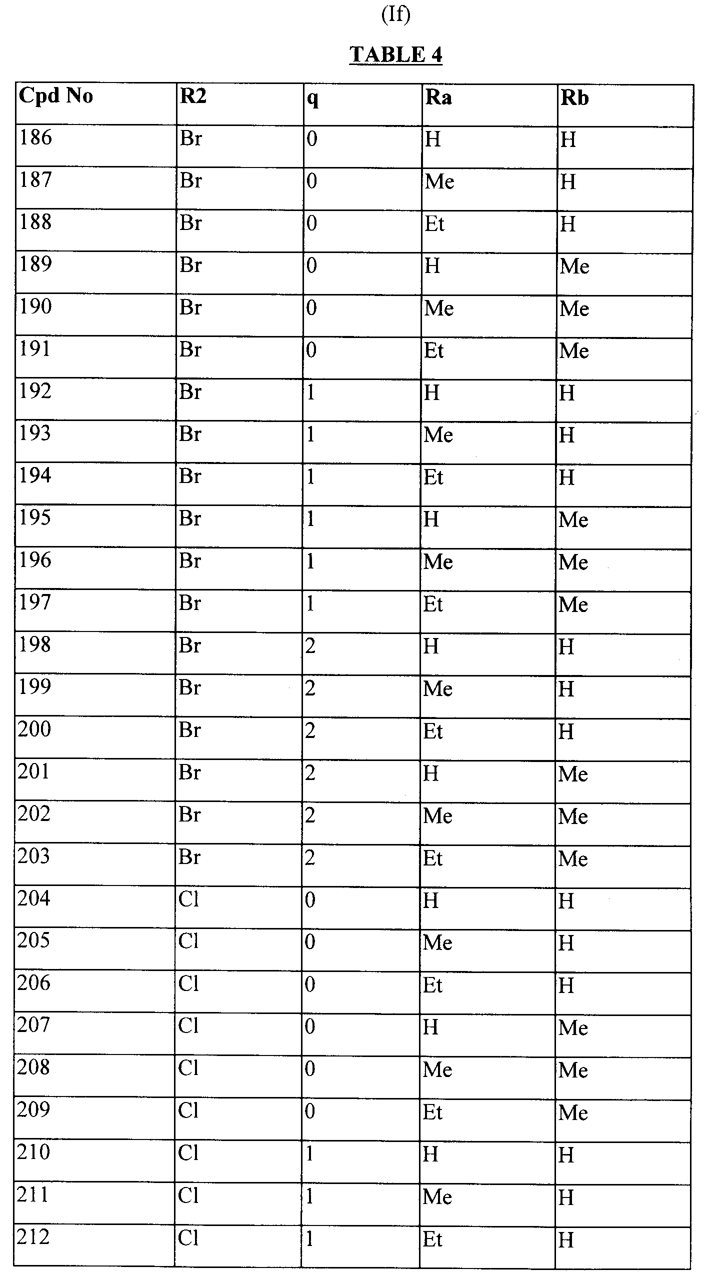

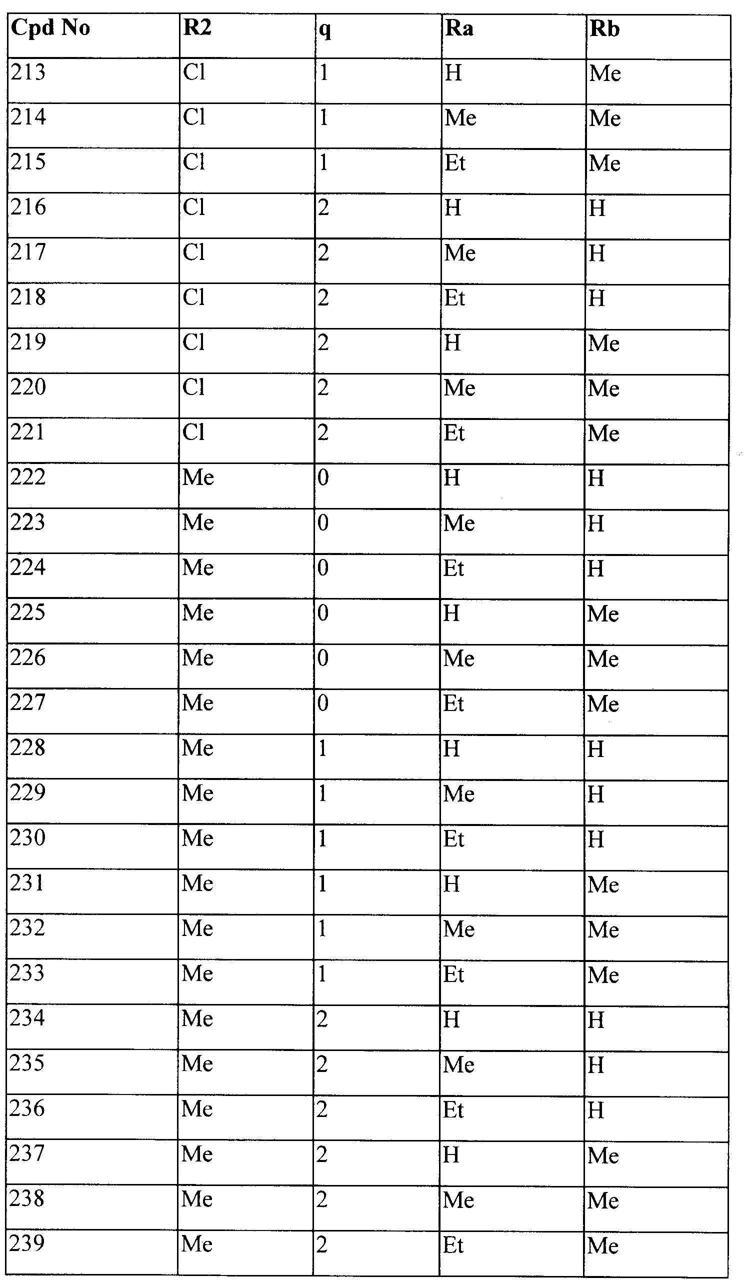

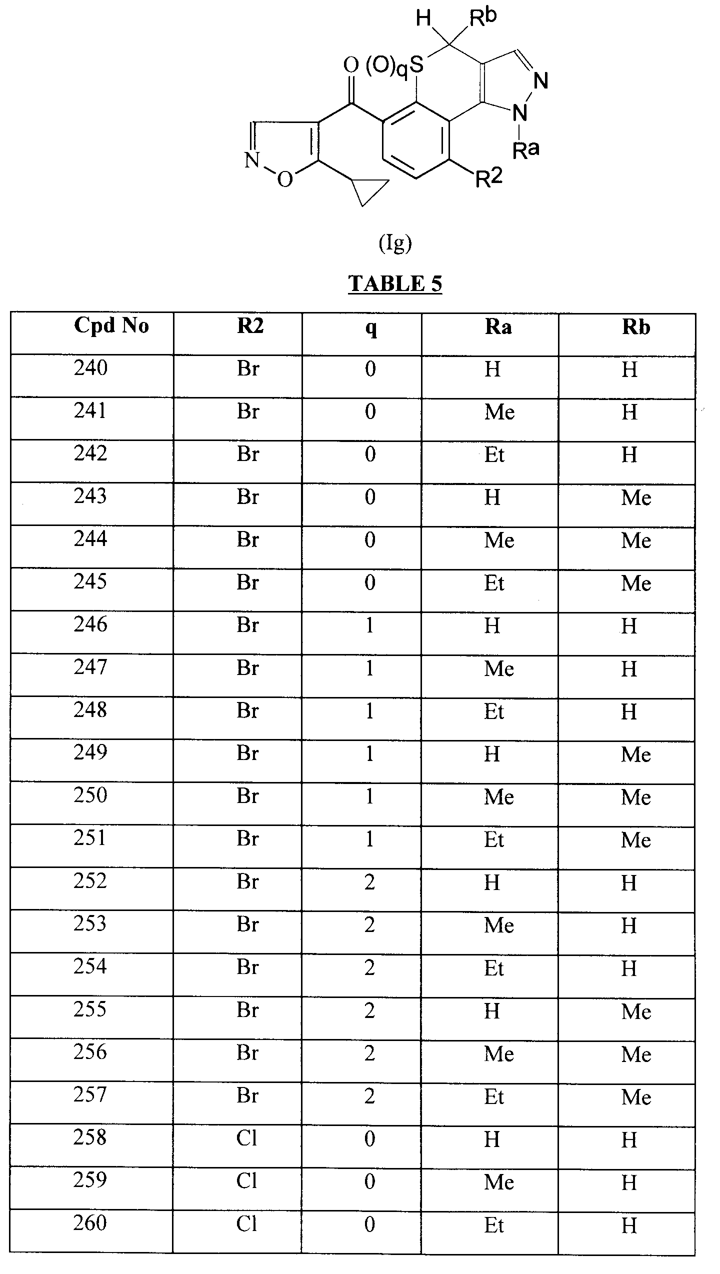

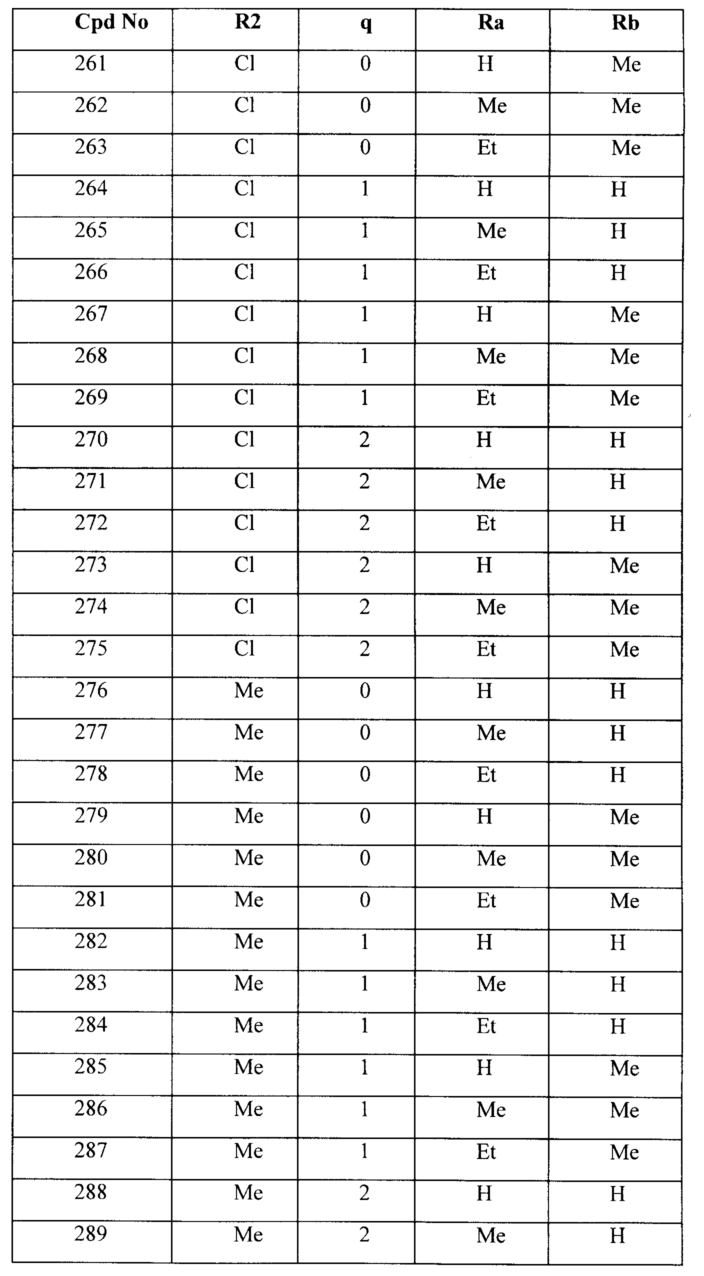

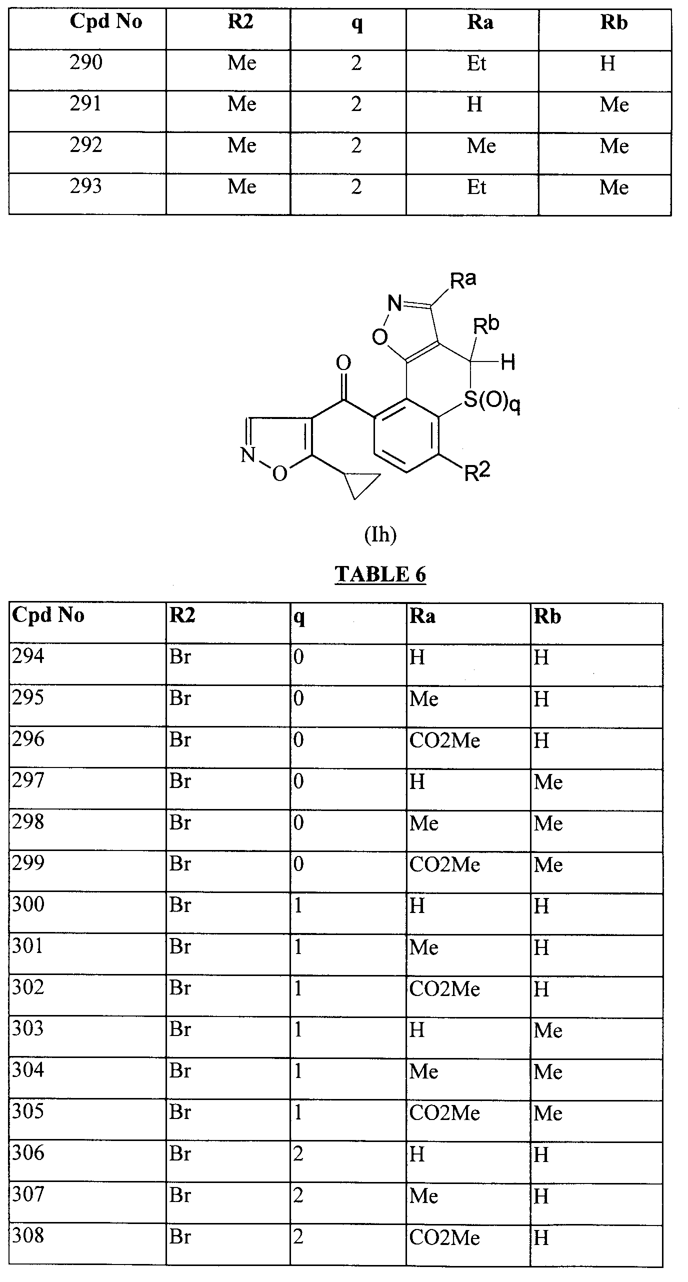

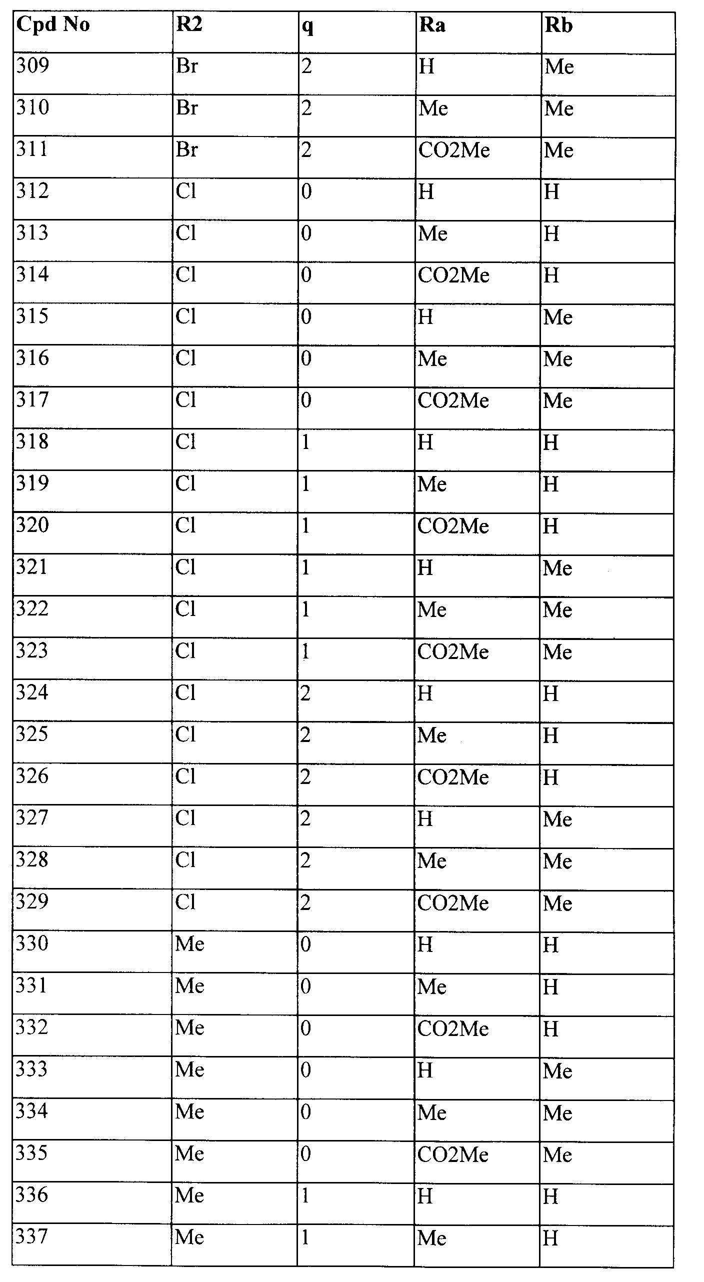

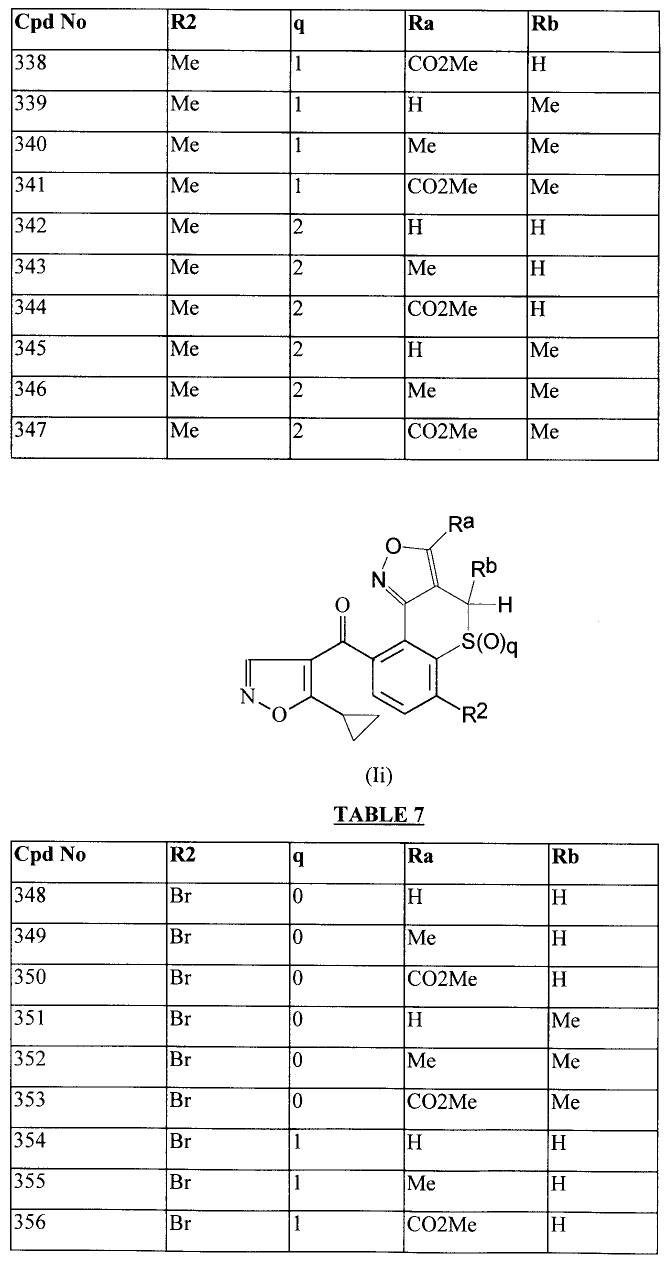

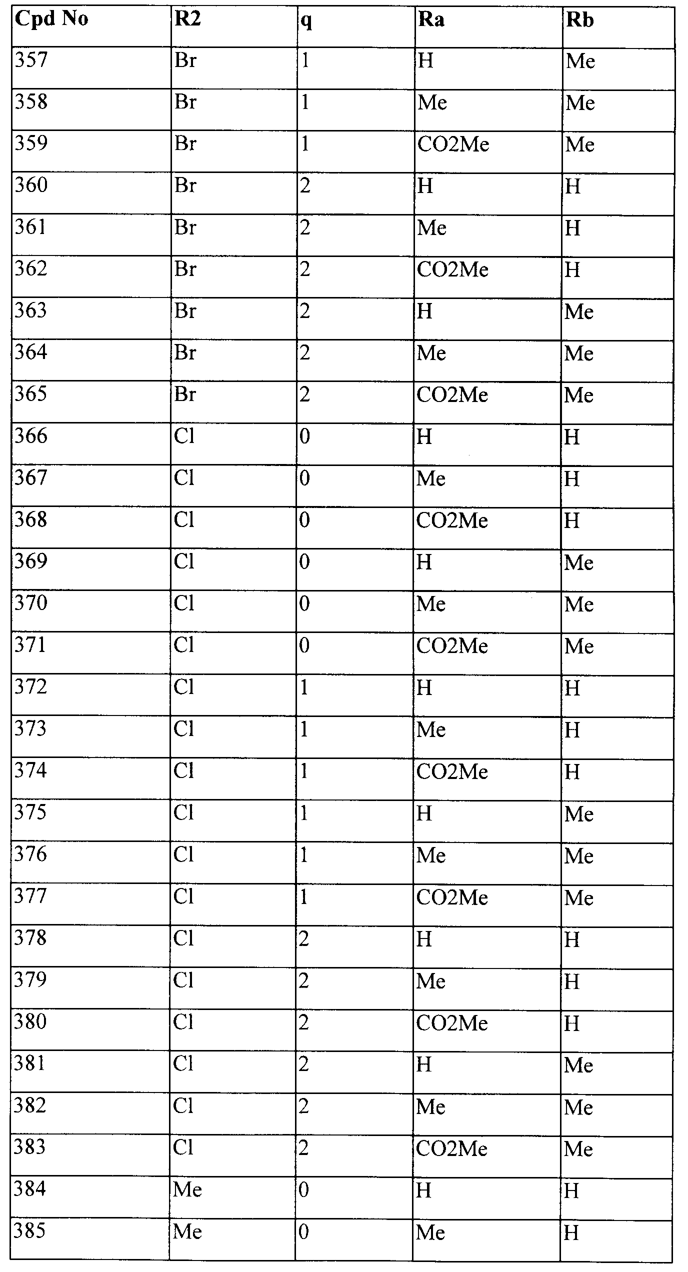

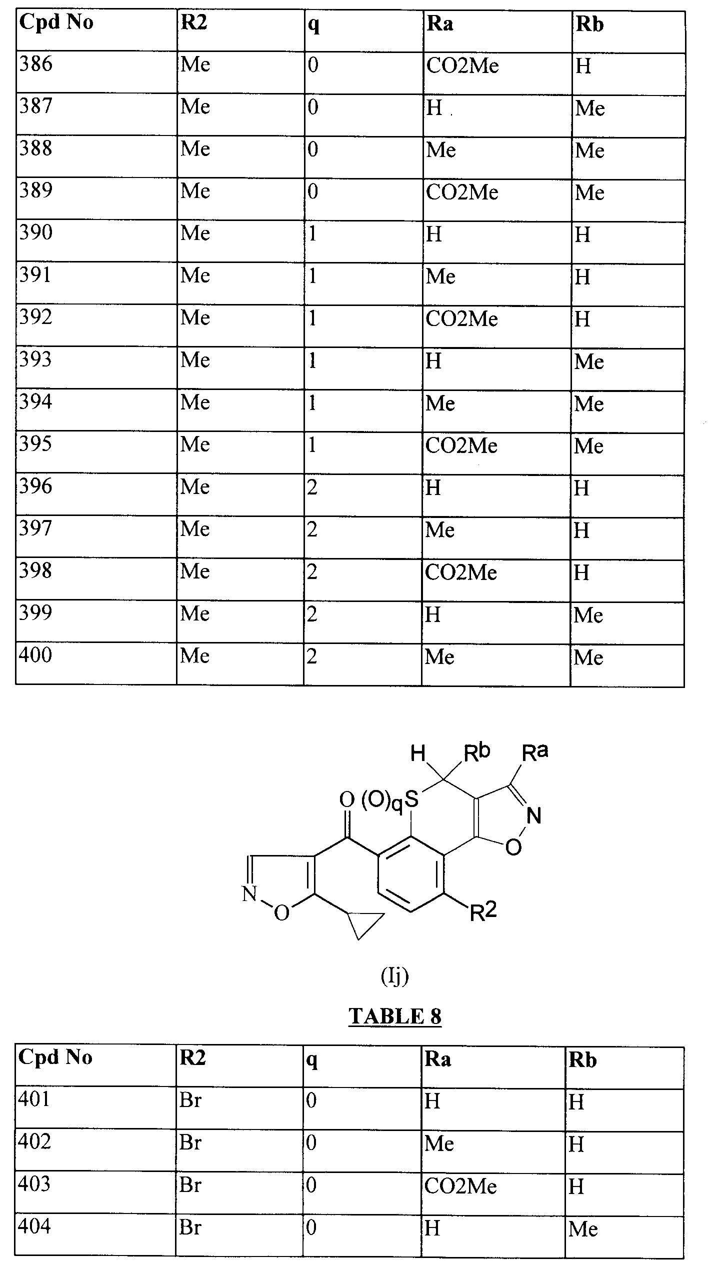

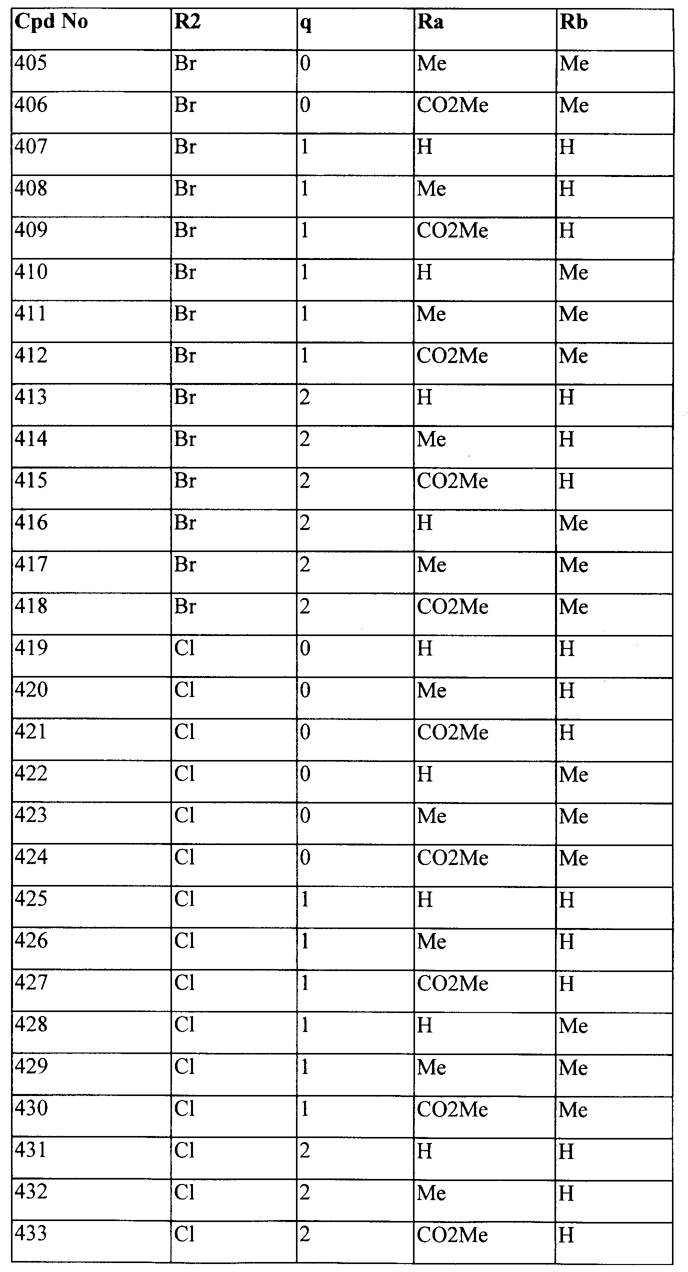

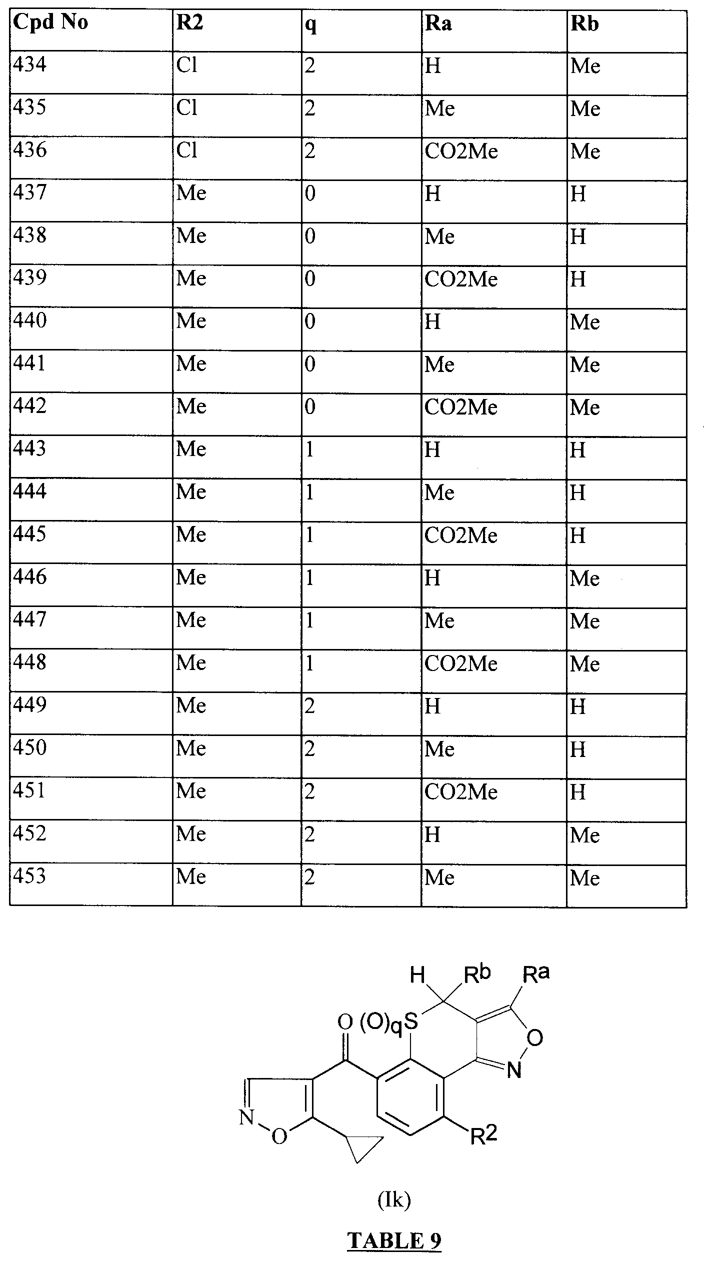

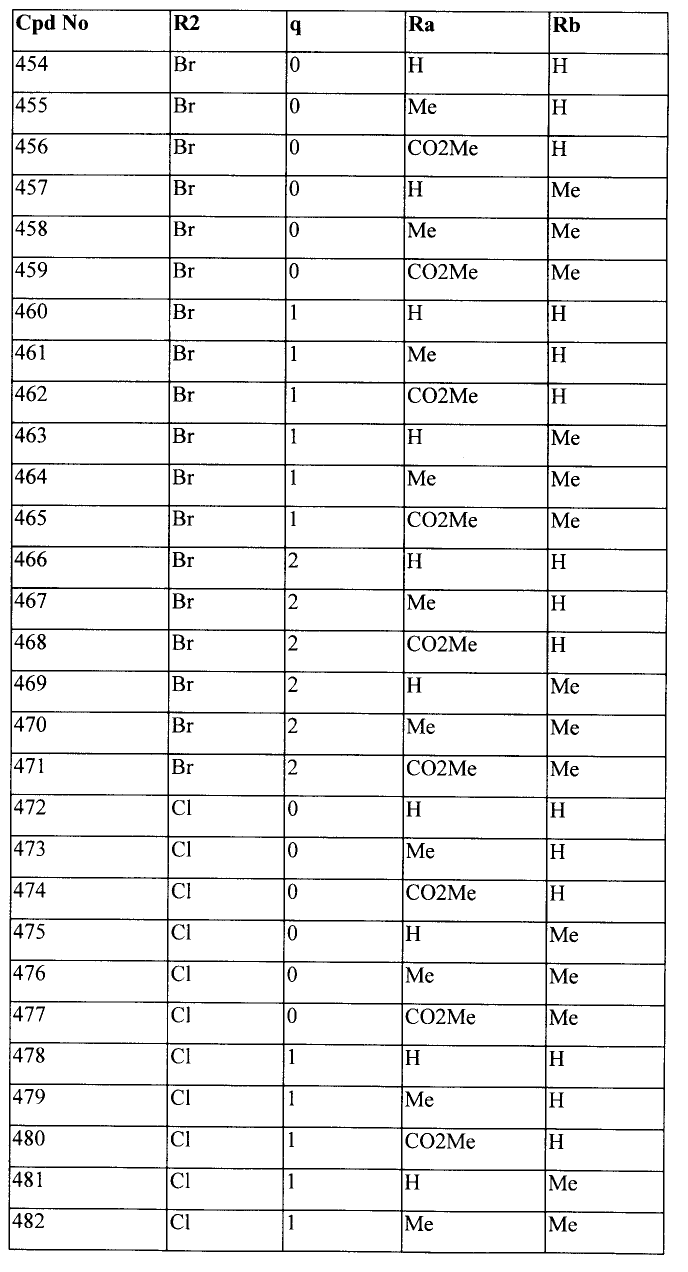

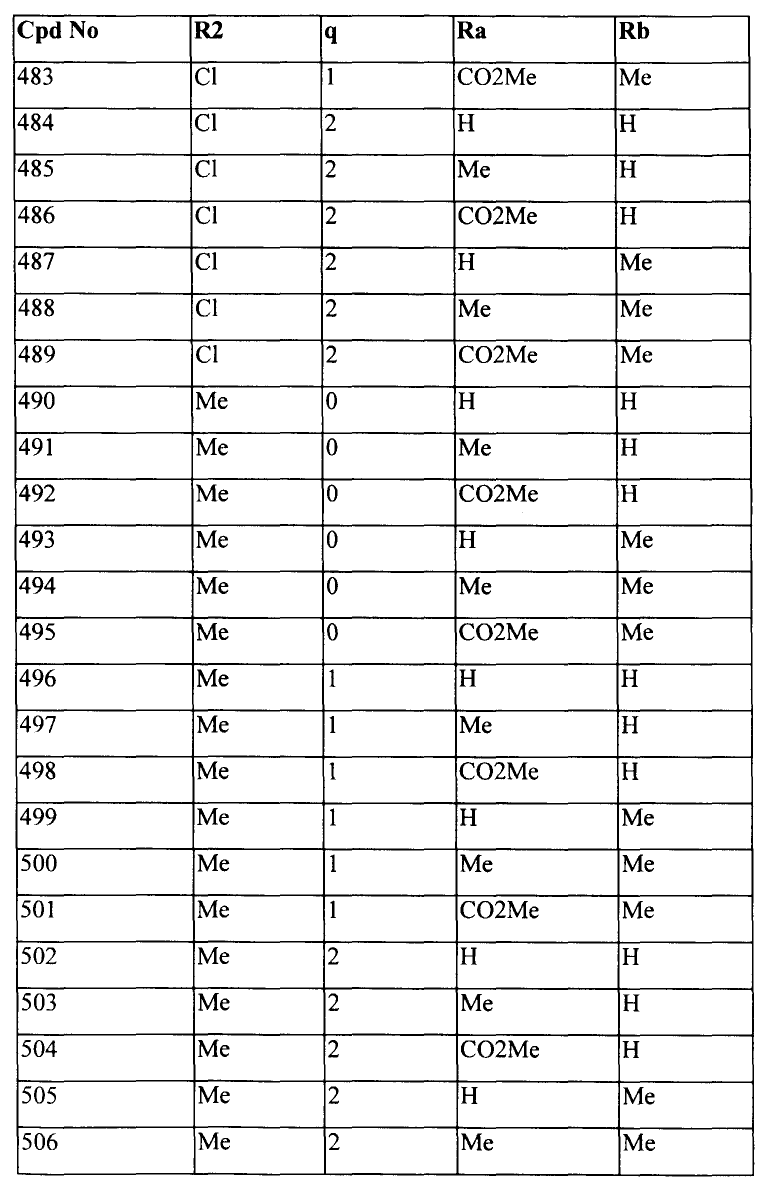

Compounds of formula (I) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders, and that suitable protecting groups may be required to achieve the compounds sought.

According to a feature of the present invention compounds of formula (Ia) in which R represents hydrogen and R¹, R², X, Y and Z are as defined above may be prepared by the reaction of a compound of formula (II): 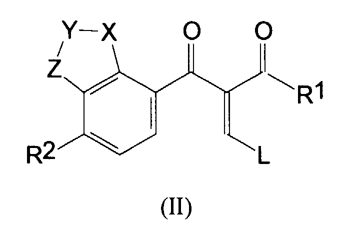 wherein L is a leaving group and R¹, R², X, Y and Z are as hereinbefore defined, with hydroxylamine or a salt of hydroxylamine. Hydroxylamine hydrochloride is generally preferred. Generally L is alkoxy, for example ethoxy, or N,N-dialkylamino, for example dimethylamino. The reaction is generally carried out in an organic solvent such as ethanol or acetonitrile or a mixture of a water-miscible organic solvent and water, preferably in a ratio of organic solvent: water of from 1:99 to 99:1, optionally in the presence of a base or acid acceptor such as triethylamine or sodium acetate at a temperature from room temperature to the boiling point of the solvent. Compounds of formula (II) are novel and as such constitute a further feature of the present invention.

According to a further feature of the present invention compounds of formula (Ia) in which R represents hydrogen and R¹, R², X, Y and Z are as defined above may be prepared by the reaction of a compound of formula (III): 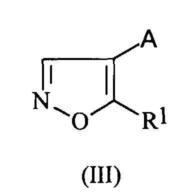 wherein R¹ is as hereinbefore defined and A represents a carboxy group or a reactive derivative thereof (such as a carboxylic acid chloride or carboxylic ester), or a cyano group, with an appropriate organometallic reagent such as a Grignard reagent or an organolithium reagent. The reaction is generally carried out in an inert solvent such as ether or tetrahydrofuran at a temperature from 0°C to the reflux temperature of the mixture.

According to a further feature of the present invention compounds of formula (Ia) wherein R represents -CO₂R³ and R¹, R², X, Y and Z are as defined above, may be prepared by the reaction of a salt of a compound of formula (IV): 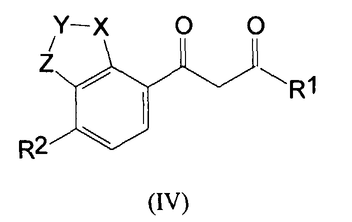 wherein R¹, R², X, Y and Z are as hereinbefore defined with a compound of formula R³O₂CC(E)=NOH wherein R³ is as hereinbefore defined and E represents halogen preferably chlorine. Preferred salts include sodium or magnesium salts. The reaction is generally performed in an inert solvent such as dichloromethane or acetonitrile at a temperature between room temperature and the reflux temperature of the mixture. The salt of a compound of formula (IV) is generally prepared in-situ by treating the compound of formula (IV) with a base. Examples of suitable bases include alkaline earth metal alkoxides such as magnesium methoxide. Compounds of formula (IV) are novel and therefore constitute a further feature of the present invention.

According to a further feature of the present invention compounds of formula (Ia) wherein R, R¹ and R² are as defined above, Y represents (CH₂)ₚ optionally substituted by lower alkyl, and one of X and Z represents S and the other represents O, may be prepared by the reaction of the corresponding compound of formula (V) or (VI): 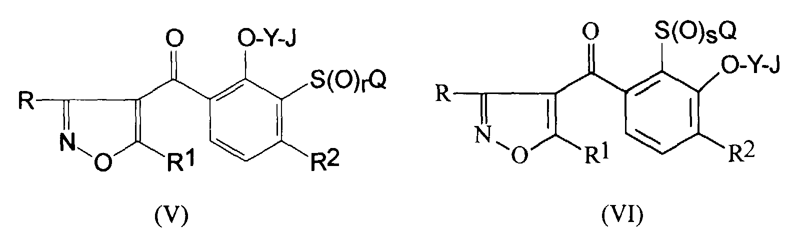 wherein R, R¹ and R² are as defined above, Y represents (CH₂)ₚ optionally substituted by lower alkyl, Q represents lower alkyl preferably methyl and J represents a leaving group generally halogen and preferably chlorine or fluorine, and r and s represent one, with an anhydride preferably trifluoroacetic anhydride in an inert solvent for example dichloromethane at a temperature of from 20°C to the reflux temperature of the solvent. The reaction proceeds via thiol intermediates of formula (V) or (VI) in which Q is replaced by hydrogen, which are generally not isolated and cyclise directly to the above compounds of formula (Ia).

According to a further feature of the present invention compounds of formula (Ia) wherein R, R¹ and R² are as defined above, Y represents (CH₂)ₚ optionally substituted by lower alkyl, and one of X and Z represents S and the other represents O, may also be prepared by cyclisation of the corresponding compound of formula (V) or (VI) wherein R, R¹ and R² are as defined above, Q represents lower alkyl preferably methyl, J represents a leaving group generally halogen and preferably chlorine or fluorine, and r and s represent zero. The reaction is generally performed in an inert solvent preferably an aprotic solvent for example N,N-dimethylformamide at a temperature of from 50°C to the reflux temperature of the solvent, preferably 100-130°C.

According to a further feature of the present invention compounds of formula (Ib) may be prepared from the corresponding compound of formula (Ia) in which R is as defined above. Where R represents a hydrogen atom the reaction is preferably carried out by treatment with a base. Examples of suitable bases include alkali or alkaline earth metal hydroxides, alkoxides such as sodium ethoxide or organic bases such as triethylamine. Where R represents -CO₂R³ the conversion is generally carried out by a hydrolytic reaction. The hydrolytic reaction may be performed in the presence of an acid or base. Acidic hydrolysis may be achieved for example using aqueous hydrochloric acid. Basic hydrolysis may be achieved for example using sodium hydroxide in a mixture of alcohol and water. The reactions are preferably carried out at a temperature between 20°C and the reflux temperature of the mixture.

According to a further feature of the present invention, compounds of formula (Ib) in which R¹, R², X, Y and Z are as defined above may also be prepared by the reaction of an acid chloride of formula (VII): 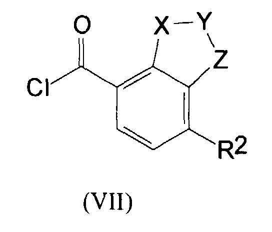 wherein R², X, Y and Z are as hereinbefore defined, with a beta-ketonitrile of formula (VIII):

R¹C(=O)CH₂CN (VIII)

wherein R¹ is as hereinbefore defined. The reaction is generally performed in the presence of a base, in a solvent or solvent mixture. Suitable bases include metal hydrides, hydroxides or alkoxides (e.g. sodium or lithium hydride, sodium hydroxide, potassium hydroxide, magnesium ethoxide or magnesium methoxide). Suitable solvents include for example tetrahydrofuran; hydrocarbons such as toluene; or halogenated hydrocarbons such as dichloromethane. The reaction is generally performed at a temperature from 0°C to the reflux temperature.

According to a further feature of the present invention, compounds of formula (Ib) in which R¹, R², X, Y and Z are as defined above may also be prepared by the reaction of an acid chloride of formula R¹COCl wherein R¹ is as hereinbefore defined, with a beta-ketonitrile of formula (IX): 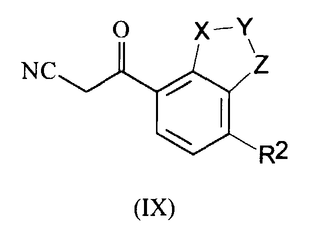 wherein R², X, Y and Z are as hereinbefore defined. The reaction is generally performed under the same conditions as described above for the reaction of compounds of formula (VII) with compounds of formula (VIII).

According to a further feature of the present invention compounds of formula (Ib) in which R¹, R², X, Y and Z are as defined above may also be prepared by the reaction of a carboxylic acid chloride of formula (VII) above wherein R², X, Y and Z are as hereinbefore defined, with a beta-ketonitrile of formula (VIII) wherein R¹ is as hereinbefore defined, via an intermediate of formula (X): 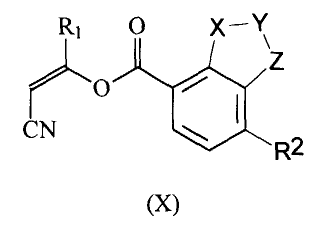 wherein R¹, R², X, Y and Z are as hereinbefore defined. The formation of the intermediate of formula (X) may be carried out in the presence of a mild base such as an organic base e.g. triethylamine, in an inert solvent such as acetonitrile or dichloromethane at a temperature between room temperature and the reflux temperature of the mixture. The rearrangement of the intermediate of formula (X) to a compound of formula (Ib) is generally carried out in situ in an inert solvent such as acetonitrile or dichloromethane in the presence of a catalyst such as a source of cyanide. Examples of such sources of cyanide are acetone cyanohydrin or an alkali metal cyanide such as potassium cyanide, optionally in the presence of a crown ether such as 18-crown-6.

According to a further feature of the present invention compounds of formula (Ib) in which R¹, R², X, Y and Z are as defined above, may be prepared by the reaction of an acid chloride of formula R¹COCl wherein R¹ is as hereinbefore defined, with a beta-ketonitrile of formula (IX) wherein R², X, Y and Z are as hereinbefore defined via an intermediate of formula (XI): 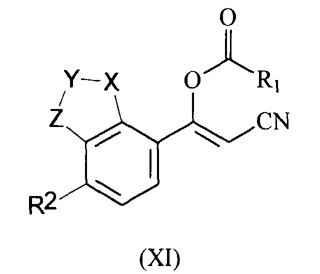 wherein R¹, R², X, Y and Z are as hereinbefore defined. The formation and rearrangement of the intermediate of formula (XI) is generally carried out under the same conditions as described above for the formation and rearrangement of compounds of formula (X).

According to a further feature of the present invention compounds of formula (I) in which R, R¹, R², X, Y and Z are as defined above wherein one or more of the sulphur atoms is at the sulphoxide or sulphone oxidation state may be prepared by the oxidation of the sulphur atom of the corresponding compounds of formula (I) in which the sulphur atom to be oxidised is at a lower oxidation state. The oxidation of the sulphur atom is generally carried out using for example 3-chloroperoxybenzoic acid in an inert solvent such as dichloromethane at a temperature from -40°C to room temperature.

Intermediates in the preparation of compounds of formula (Ia) and (Ib) may be prepared by the application or adaptation of known methods.

Compounds of formula (II) may be prepared by the reaction of compounds of formula (IV) with either a trialkyl orthoformate such as triethyl orthoformate or a dimethylformamide dialkyl acetal such as dimethylformamide dimethyl acetal.

The reaction with a trialkyl orthoformate can be carried out in the presence of acetic anhydride at the reflux temperature of the mixture and the reaction with dialkylformamide dialkyl acetal can be carried out optionally in the presence of an inert solvent at a temperature from room temperature to the reflux temperature of the mixture.

Compounds of formula (IV) may be prepared by the reaction of an acid chloride of formula (VII) with a metal salt of a compound of formula (XII): 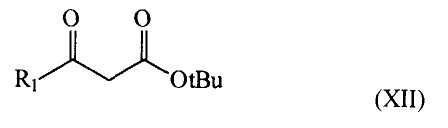 wherein R¹ is as hereinbefore defined, to give a compound of formula (XIII): 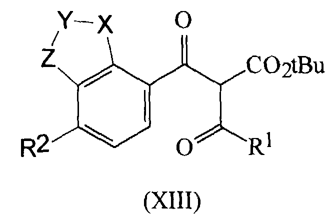 wherein R¹, R², X, Y and Z are as hereinbefore defined, which is subsequently decarboxylated to give a compound of formula (IV). Generally the reaction to produce the compound of formula (XIII) is performed in a solvent such as a lower alcohol, preferably methanol, in the presence of a metal, preferably magnesium. The reaction may also be performed using a pre-prepared metal salt of a compound of formula (XII). The decarboxylation is generally performed by refluxing the compound of formula (XIII) in the presence of a catalyst, such as para-toluenesulphonic acid or trifluoroacetic acid, in an inert solvent e.g. toluene or 1,2-dichloroethane.

Compounds of formula (IV) may also be prepared by the reaction of a carboxylic acid ester of formula (XIV): 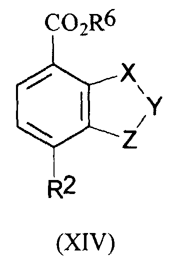 wherein R², X, Y and Z are as hereinbefore defined and R⁶ represents a lower alkyl group (preferably methyl), with a compound of formula (XV):

R¹-C(O)-CH₃ (XV)

wherein R¹ is as hereinbefore defined. The reaction is generally performed in a solvent such as ether, tetrahydrofuran or N,N-dimethylformamide, in the presence of a base, preferably an alkali metal base such as sodium hydride, at a temperature from 0°C to the reflux temperature. Compounds of formula (XIV) are novel and as such constitute a further feature of the present invention.

Intermediates of formula (V) and (VI) may be prepared by any of the processes described above for the preparation of a compound of formula (Ia) from the corresponding compounds of formula (II), (III) or (IV).

Acid chlorides of formula (VII) may be prepared by the reaction of a benzoic acid of formula (XVI): 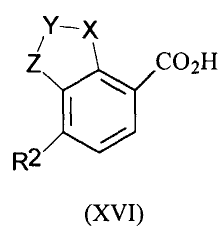 with a chlorinating agent, for example thionyl chloride at the reflux temperature of the mixture. In some cases the benzoyl chlorides may also be prepared by reaction of the benzoic acid with oxalyl chloride in a solvent such as 1,2-dichloroethane at from ambient to reflux temperature.

Carboxylic acids of formula (XVI) wherein R² is as defined above, Y represents (CH₂)_{P} optionally substituted by lower alkyl, and one of X and Z represents S and the other O, may be prepared by the hydrolysis of a compound of formula (XVII) or (XVIII): 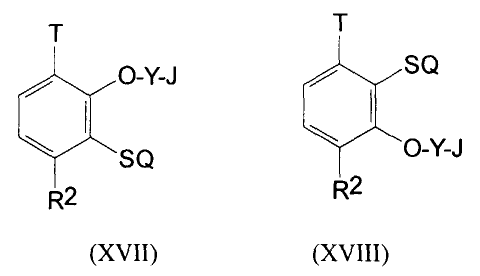 wherein T represents a protected carboxy group which is converted into the carboxy group during the hydrolysis, J represents a leaving group generally halogen for example fluorine or chlorine, and Q represents lower alkyl preferably methyl, with an acid for example hydrochloric acid generally at the reflux temperature. Preferred T groups include alkoxycarbonyl and 4,4-dimethyl-1,3-oxazolin-2-yl.

A number of the acids of formula (XVI) are novel and as such constitute a further feature of the present invention.

Esters of formula (XIV) may be prepared from acids of formula (XVI) according to known methods.

Beta-ketonitriles of formula (VIII) may be prepared from acid chlorides of formula R¹COCl by a number of methods well known in the chemical literature. For example, see Krauss, et al, Synthesis, **1983**, 308, or Muth, et al, J. Org. Chem, **1960**, 25, 736. Alternatively betaketonitriles of formula (VIII) may be prepared by the reaction of an ester of formula R¹-CO₂Et, wherein R¹ is as hereinbefore defined, with acetonitrile. This reaction is described in the literature, for example see the article by Abramovitch and Hauser, J.Am. Chem. Soc., **1942**, 64, 2720.

Beta-ketonitriles of formula (IX) may be prepared from acid chlorides of formula (VII) or from corresponding ethyl benzoates in a manner analogous to the preparation of beta-ketonitriles of formula (VIII) set forth above.

Intermediates of formula (III), (XII), (XV), (XVII) and (XVIII) are known or may be prepared by the application or adaptation of known methods.

Those skilled in the art will appreciate that some compounds of formula (I) may be prepared by the interconversion of other compounds of formula (I) and such interconversions constitute yet more features of the present invention.

The following non-limiting Examples illustrate the preparation of compounds of formula (I) and the Reference Examples illustrate the preparation of intermediates in their synthesis. NMR Spectra are recorded as d (ppm) in deuterochloroform as solvent (unless ortherwise stated).

### Example 1

A mixture of trifluoroacetic anhydride (3.3g) and 4-[4-bromo-2-(2-fluoroethoxy)-3-methylsulphinylbenzoyl]-5-cyclopropylisoxazole (2.0g) was heated at reflux in dichloromethane for 1.5 hours. After evaporation the residue was stirred at 70°C in a 1:1 mixture of tetrahydrofuran and water for 1.5 hours. The organic phase was dried (magnesium sulphate), evaporated and purified by chromatography on silica gel eluting with dichloromethane/hexane (3:2) followed by crystallisation (toluene/hexane) to give 4-(5-bromo-1,4-benzoxathian-8-oyl)-5-cyclopropylisoxazole (0.3g, Compound 1), mp135-136°C, NMR 1.2(m,2H), 1.3(m,2H), 2.7(m,1H), 3.2(m,2H), 4.3(m,2H), 7.0 (d,1H), 7.2(d,1H), 8.2(s,1H).

### Example 2

Sodium acetate (0.32g) and hydroxylamine hydrochloride (0.36g) were added to a stirred solution of 3-cyclopropyl-2-ethoxymethylene-1-(5-fluoro-1,4-benzoxathian-8-yl)propan-1,3-dione (1.44g) in ethanol. The mixture was stirred overnight, diluted with water, extracted (ethyl acetate), dried (magnesium sulphate) and evaporated. The residue was purified by chromatography on silica gel eluting with hexane/ethyl acetate (9:1) to give 5-cyclopropyl-4-(5-fluoro-1,4-benzoxathian-8-oyl)isoxazole (0.5g, Compound 4), NMR 1.1(m,2H), 1.2(m,2H), 2.7(m,1H), 3.0(m,2H), 4.3(m,2H), 6.7(m,1H), 7.1(m,1H), 8.1(s,1H).

By proceeding in a similar manner the following compounds were also prepared:
4-(8-chloro-1,4-benzoxathian-5-oyl)-5-cyclopropyl-isoxazole (Compound 7), mp 88-92°C, NMR 1.1(m,2H), 1.3(m,2H), 2.6(m,1H), 3.0(m,2H), 4.5(m,2H), 7.0(d,1H), 7.1(d,1H), 8.2(s,1H);
5-cyclopropyl-4-(8-methyl-1,4benzoxathian-5-oyl)isoxazole (Compound 13), NMR 1.2(m,2H), 1.3(m,2H), 2.2(s,3H), 2.7(m,1H), 3.0(m,2H), 4.5(m,3H), 6.9(d,1H), 7.1(d,1H), 8.3(s,1H);
5-cyclopropyl-4-(8-fluoro-1,4-benzoxathian-5-oyl)isoxazole (Compound 31), NMR 1.2(m,2H), 1.3(m,2H), 2.7(m,1H), 3.1(m,2H), 4.6(m,2H), 6.9(m,1H), 7.2(m,1H), 8.3(s,1H); and
5-cyclopropyl-4-(8-methylthio-1,4-benzoxathian-5-oyl)isoxazole (Compound 32), NMR 1.1-1.3(m,4H), 2.4(s,3H), 2.6(m,1H), 3.0(m,2H), 4.5(m,2H), 6.8(d,1H), 7.1(d,1H), 8.2(s,1H).

### Example 3

By proceeding in a similar manner to Example 2 there was prepared from 1-[4-bromo-3-(1-chloro-2-propyloxy)-2-methylthiophenyl]-3-cyclopropyl-2-ethoxymethylenepropan-1,3-dione the following compound in which the additional cyclisation reaction to form the 1,4-benzoxathian ring had also occurred:
5-cyclopropyl-4-(8-bromo-2-methyl-1,4-benzoxathian-5-oyl)isoxazole (Compound 6), mp 120-121°C.

By proceeding in a similar manner the following compounds were also prepared:
4-(8-bromo-3-methyl-1,4-benzoxathian-5-oyl)-5-cyclopropylisoxazole (Compound 14), mp 103-105°C (from 1-[4-bromo-3-(2-chloro-1-propyloxy)-2-methylthiophenyl]-3-cyclopropyl-2-ethoxymethylene-propan-1,3-dione); and
4-(8-bromo-1,4-benzoxathian-5-oyl)-5-cyclopropylisoxazole (Compound number 36), NMR 1.2(m,2H), 1.3(m,2H), 2.7(m,1H), 3.1(m,2H), 4.6(m,2H), 7.0(d,1H), 7.4(d,1H), 8.2(s,1H) (from 1-[4-bromo-3-(2-chloroethoxy)-2-methylthiophenyl]-3-cyclopropyl-2-ethoxymethylene-propan-1,3-dione).

### Example 4

Hydroxylamine hydrochloride (0.44 g) and sodium acetate (0.1 g) were added to a solution of 3-cyclopropyl-2-dimethylaminomethylene-1-(8-methoxy-1,4-benzoxathian-5-yl)propan-1,3-dione (1.7g) in ethanol. The mixture was stirred overnight and evaporated. Water was added and the mixture extracted with dichloromethane, dried (magnesium sulphate), evaporated and crystallised from ethanol to give 5-cyclopropyl-4-(8-methoxy-1,4-benzoxathian-5-oyl)isoxazole (1.14g, Compound 11), NMR 1.2(m,2H), 1.3(m,2H), 2.7(m,1H), 3.0(m,2H), 3.9(s,3H), 4.6(m,2H), 6.7(d,1H), 7.2(d,1H).

By proceeding in a similar manner the following compounds were also prepared:
4-(7-bromo-1,3-benzoxathiolan-4-oyl)-5-cyclopropyl-isoxazole (Compound 12), mp127-128°C;
5-cyclopropyl-4-(9-fluoro-1,5-benzoxathiepan-6-oyl)isoxazole (Compound 17), NMR 1.2(m,2H), 1.3(m,2H), 2.3(m,2H), 2.6(m,1H), 3.0(m,2H), 4.4(m,2H), 7.0(m,2H), 8.2(s,1H);
4-(5-chloro-1,4-benzoxathian-8-oyl)-5-cyclopropylisoxazole (Compound 20), NMR 1.1(m,2H), 1.2(m,2H), 2.7(m,1H), 3.1(m,2H), 4.3(m,2H), 7.0(s,2H), 8.1(s,1H); and
5-cyclopropyl-4-(5-methylthio-1,4-benzoxathian-8-oyl)isoxazole (Compound 23), NMR 1.2(m,2H), 1.3(m,2H), 2.5(s,3H), 2.8(m,1H), 3.2(m,2H), 4.4(m,2H), 6.9(d,1H), 7.1(d,1H), 8.2(s,1H).

### Example 5

m-Chloroperbenzoic acid (25mg) was added to a stirred solution of 4-(5-bromo-1,4-benzoxathian-8-oyl)-5-cyclopropylisoxazole (50mg) in dichloromethane at 20°C. After 3 hours the mixture was washed with sodium metabisulphite solution and sodium carbonate solution. The organic layer was dried (magnesium sulphate) and evaporated to give 4-(5-bromo-1,4-benzoxathian-8-oyl)-5-cyclopropylisoxazole S,S-dioxide (35mg, Compound 2), mp190-193°C.

By proceeding in a similar manner the following compounds were also prepared:
4-(5-bromo-1,4-benzoxathian-8-oyl)-5-cyclopropylisoxazole S-oxide (Compound 3), mp166-168°C;
5-cyclopropyl-4-(8-methyl-1,4-benzoxathian-5-oyl)isoxazole S-oxide (Compound 5), NMR 1.1-1.3(m,4H), 2.2(s,3H), 2.9(m,3H), 4.7-4.8(m,2H), 7.1(d,1H), 7.3(d,1H), 8.2(s,1H).
4-(8-chloro-1,4-benzoxathian-5-oyl)-5-cyclopropylisoxazole S-oxide (Compound 8),NMR 1.1-1.4(m,4H), 2.8(m,1H), 3.1(m,2H), 4.7(m,2H), 7.1(d,1H), 7.6(d,1H), 8.1(s,1H);
4-(8-methoxy-1,4-benzoxathian-5-oyl)-5-cyclopropylisoxazole S,S-dioxide (Compound 9),NMR 1.2(m,2H), 1.3(m,2H), 2.8(m,1H), 3.4(m,2H), 4.0(s,3H), 5.0(m,2H), 7.0(d,1H), 7.1(d,1H), 8.2(s,1H);
4-(8-methoxy-1,4-benzoxathian-5-oyl)-5-cyclopropylisoxazole S-oxide (Compound 10), NMR 1.1-1.3(m,4H), 3.0(m,3H), 3.9(s,3H), 4.8(m,2H), 7.0(d,1H), 73(d,1H), 8.2(s,1H);
5-cyclopropyl-4-(9-fluoro-1,5-benzoxathiepan-6-oyl)isoxazole S,S-dioxide (Compound 15), NMR 1.2(m,2H), 1.3(m,2H), 2.4(m,2H), 2.6(m,1H), 3.4(m,2H), 4.4(m,2H), 7.1(m,1H), 7.4(t,1H), 8.2(s,1H);
5-cyclopropyl-4-(9-fluoro-1,5-benzoxathiepan-6-oyl)isoxazole S-oxide (Compound 16), NMR 1.2(m,2H), 1.3(m,2H), 2.4(m,1H), 2.7(m,1H),2.9(m,1H),3.1(m,1H),3.5(m,1H),4.3(m,1H),4.6(m,1H), 7.2-7.3(m,2H), 8.2(s,1H);
4-(5-chloro-1,4-benzoxathian-8-oyl)-5-cyclopropylisoxazole S,S-dioxide (Compound 18), NMR 1.2(m,2H), 1.3(m,2H), 2.6(m,1H), 3.6(m,2H), 4.7(m,2H), 7.2(d,1H), 7.4(d,1H), 8.1(s,1H);
4-(5-chloro-1,4-benzoxathian-8-oyl)-5-cyclopropylisoxazole S-oxide (Compound 19), NMR 1.1-1.3(m,4H), 2.6(m,1H), 3.1(m,2H), 4.6(m,2H), 7.1(d,1H), 7.5(d,1H), 8.1(s,1H);
5-cyclopropyl-4-(5-methylsulphonyl-1,4-benzoxathian-8-oyl)isoxazole S,S-dioxide (Compound 21), NMR 1.1-1.3(m,4H), 2.6(m,1H), 3.3(s,3H), 3.5(m,2H), 4.8(m,2H), 7.6(d,1H), 7.9(d,1H), 8.1(s,1H);
5-cyclopropyl-4-(5-methylthio-1,4-benzoxathian-8-oyl)isoxazole S-oxide (Compound 22), NMR 1.1-1.3(m,4H), 2.5(s,3H), 2.6(m,1H), 2.9(m,1H), 3.1(m,1H), 4.5(m,2H), 7.0(d,1H), 7.5(d,1H), 8.1(s,1H);
ethyl 5-cyclopropyl-4-(8-fluoro-1,4-benzoxathian-5-oyl)isoxazole-3-carboxylate S,S-dioxide (Compound 24), NMR 1.2(t,3H), 1.2-1.4(m,4H), 2.6(m,1H), 3.5(m,2H), 4.1(q,2H), 5.0(m,2H), 7.1(m,1H), 7.2(m,1H);
ethyl 5-cyclopropyl-4-(8-fluoro-1,4-benzoxathian-5-oyl)isoxazole-3-carboxylate S-oxide (Compound 25), NMR 1.1(t,3H), 1.1-1.3(m,4H), 2.4(m,1H), 2.9(m,1H), 3.2(d,1H), 4.0(q,2H), 4.7(m,1H), 4.9(m,1H), 7.1(m,1H), 7.2(m,1H);
ethyl 5-cyclopropyl-4-(8-methylsulphonyl-1,4-benzoxathian-5-oyl)isoxazole-3-carboxylate S,S-dioxide (Compound 28), NMR 1.2(t,3H), 1.2-1.5(m,4H), 2.7(m,1H), 3.2(s,3H), 3.6(m,2H), 4.1(q,2H), 5.1(m,2H), 7.2(d,1H), 8.2(d,1H)(from ethyl 5-cyclopropyl-4-(8-methylthio-1,4-benzoxathian-5-oyl)isoxazole-3-carboxylate);
5-cyclopropyl-4-(8-fluoro-1,4-benzoxathian-5-oyl)isoxazole S,S-dioxide (Compound 29), NMR 1.2(m,2H), 1.4(m,2H), 2.8(m,1H), 3.5(m,2H), 5.0(m,2H), 7.1(m,1H), 7.3(m,1H), 8.2(s,1H);
5-cyclopropyl-4-(8-fluoro-1,4-benzoxathian-5-oyl)isoxazole S-oxide (Compound 30), NMR 1.2-1.4(m,4H), 2.9(m,1H), 3.1(m,2H), 4.8(m,2H), 7.2-7.4(m,2H), 8.2(s,1H);
5-cyclopropyl-4-(8-methylsulphonyl-1,4-benzoxathian-5-oyl)isoxazole S,S-dioxide (Compound 33), NMR 1.2(m,2H), 1.3(m,2H), 2.7(m,1H), 3.2(s,3H), 3.5(m,2H), 5.1(m,2H), 7.1(d,1H), 8.1(s,1H), 8.2(d,1H) (from 5-cyclopropyl-4-(8-methylthio-1,4-benzoxathian-5-oyl)isoxazole);
4-(8-bromo-1,4-benzoxathian-5-oyl)-5-cyclopropylisoxazole S,S-dioxide (Compound 34), mp204-206, NMR 1.2(m,2H), 1.4(m,2H), 2.8(m,1H), 3.5(m,2H), 5.0(m,2H), 7.0(d,1H), 7.8(d,1H), 8.2(s,1H);
4-(8-bromo-1,4-benzoxathian-5-oyl)-5-cyclopropylisoxazole S-oxide (Compound 35), mp144-146, NMR 1.2-1.4(m,4H), 2.9(m,1H), 3.1(m,2H), 4.9(m,2H), 7.1(d,1H), 7.8(d,1H), 8.2(s,1H); and
5-cyclopropyl-4-(8-methyl-1,4-benzoxathian-5-oyl)isoxazole S,S-dioxide (Compound 38), NMR 1.2(m,2H), 1.3(m,2H), 2.3(s,3H), 2.8(m,1H), 3.4(m,2H), 5.0(m,2H), 7.0(d,1H), 7.4(d,1H), 8.2(s,1H).

### Example 6

A solution of 3-cyclopropyl-1-(8-fluoro-1,4-benzoxathian-5-yl)-propan-1,3-dione (15.1g) in methanol was added to a stirred solution of magnesium (1.36g) in methanol. The mixture was heated at reflux for 0.5 hour and evaporated. Toluene was added and re-evaporated. Ethyl chloro-oximidoacetate (9.6g) was added to a solution of the residue in dichloromethane and stirred at 20°C for 4 days. Hydrochloric acid (2M) was added with stirring and the organic phase washed (water), dried (magnesium sulphate), evaporated and purified by chromatography on silica gel eluting with ethyl acetate/hexane (1:4) to give ethyl 5-cyclopropyl-4-(8-fluoro-1,4-benzoxathian-5-oyl)isoxazole-3-carboxylate (10.1g, Compound 26), NMR 1.1(t,3H), 1.1-1.3(m,4H), 2.2(m,1H), 3.1(m,2H), 4.2(q,2H), 4.5(m,2H), 6.8(m,1H), 7.1(m,1H).

### Example 7

Sodium thiomethoxide (0.045g) was added to a solution of ethyl 5-cyclopropyl-4-(8-fluoro-1,4-benzoxathian-5-oyl)isoxazole-3-carboxylate (0.2g) in N,N-dimethylformamide and stirred at 20°C overnight. After evaporation the residue was purified by chromatography on silica gel eluting with ethyl acetate/hexane (1:4) to give ethyl 5-cyclopropyl-4-(8-methylthio-1,4-benzoxathian-5-oyl)isoxazole-3-carboxylate (0.16g, Compound 27), NMR 1.1(t,3H), 1.1-1.3(m,4H), 2.2(m,1H), 2.4(s,3H), 3.1(m,2H), 4.1(q,2H), 4.6(m,2H), 6.8(d,1H), 7.1(d,1H).

### Example 8

A mixture of 4-(8-bromo-1,4-benzoxathian-5-oyl)-5-cyclopropylisoxazole S-oxide (0.1 g) and triethylamine (0.27ml) was stirred in dichloromethane for 18 hours, washed with hydrochloric acid (2M) and water and the organic phase dried (magnesium sulphate) and evaporated to give 1-(8-bromo-1,4-benzoxathian-5-yl)-2-cyano-3-cyclopropyl-propan-1,3-dione S-oxide (0.086g, Compound 37), mp163-165°C.

### Reference Example 1

By proceeding in a similar manner to Example 5 the following compound was also prepared:
4-[4-bromo-2-(2-fluoroethoxy)-3-methylsulphinylbenzoyl]-5-cyclopropylisoxazole, mp115-117°C.

### Reference Example 2

By proceeding in a similar manner to Example 2 the following compound was also prepared:
4-[4-bromo-2-(2-fluoroethoxy)-3-(methylthio)benzoyl]-5-cyclopropylisoxazole, mp71-74°C.

### Reference Example 3

A mixture of 1-[4-bromo-2-(2-fluoroethoxy)-3-methylthiophenyl]-3-cyclopropylpropan-1,3-dione (6.0g) and triethylorthoformate (5.2g) in acetic anhydride (100 ml) was heated under reflux for 1.5 hours. The mixture was evaporated to give 1-[4-bromo-2-(2-fluoroethoxy)-3-methylthiophenyl]-3-cyclopropyl-2-ethoxymethylene-propan-1,3-dione (7.2g) as an orange oil, which was used directly in the next stage. By proceeding in a similar manner the following compounds were also prepared:
1-[3-(1-chloro-2-propyloxy)-4-bromo-2-methylthiophenyl]-3-cyclopropyl-2-ethoxymethylene-propan-1,3-dione;
3-cyclopropyl-2-ethoxymethylene-1-(5-fluoro-1,4-benzoxathian-8-yl)propan-1,3-dione;
3-cyclopropyl-2-ethoxymethylene-1-(8-chloro-1,4-benzoxathian-5-yl)propan-1,3-dione;
3-cyclopropyl-2-ethoxymethylene-1-(8-fluoro-1,4-benzoxathian-5-yl)propan-1,3-dione;
3-cyclopropyl-2-ethoxymethylene-1-(8-methylthio-1,4-benzoxathian-5-yl)propan-1,3-dione;
1-[4-bromo-3-(2-chloroethoxy)-2-methylthiophenyl]-3-cyclopropyl-2-ethoxymethylene-propan-1,3-dione;
3-cyclopropyl-2-ethoxymethylene-1-(8-methyl-1,4-benzoxathian-5-yl)propan-1,3-dione; and
1-[4-bromo-3-(2-chloro-1-propyloxy)-2-methylthiophenyl]-3-cyclopropyl-2-ethoxymethylene-propan-1,3-dione.

### Reference Example 4

A mixture of t-butyl 3-cyclopropyl-3-oxopropionate methoxymagnesium enolate (1.7g) and 5-fluoro-1,4-benzoxathian-8-carboxylic acid chloride (1.3g) was stirred in toluene at 20°C overnight. Hydrochloric acid (2M) was added and stirred for 1 hour. The organic layer was dried (magnesium sulphate), 4-toluenesulphonic acid (0.19g) added and heated at reflux for 1.5 hours. The solution was washed (water), dried (magnesium sulphate) and evaporated to give 3-cyclopropyl-1-(5-fluoro-1,4-benzoxathian-8-yl)propan-1,3-dione (1.2g), NMR 0.9(m,2H), 1.2(m,2H), 1.8(m,1H), 3.2(m,2H), 4.5(m,2H), 6.7(t,1H), 7.6(m,1H), 16.5.

By proceeding in a similar manner the following compounds were also obtained:
1-[4-bromo-2-(2-fluoroethoxy)-3-methylthiophenyl]-3-cyclopropylpropan-1,3-dione, NMR 0.95(m,2H), 1.15(m,2H), 1.8(m,1H), 2.45(s,3H), 4.15(m,1H), 4.25(m,1H), 4.6(m,1H), 4.8(m,1H), 6.6(s,1H), 7.5(m,2H), 14.0(s,1H);
1-[4-bromo-3-(1-chloro-2-propyloxy)-2-methylthiophenyl]-3-cyclopropylpropan-1,3-dione, NMR 0.87(m,2H), 1.2(m,2H), 1.47(d,3H), 1.75(m,1H), 2.4(s,3H), 3.75(t,2H), 4.9(m,1H), 5.9(s,1H), 7.1(d,1H), 7.5(d,1H), 15.9(bs,1H);
1-(8-chloro-1,4-benzoxathian-5-yl)]-3-cyclopropylpropan-1,3-dione, NMR 1.0(m,2H), 1.2(m,2H), 1.7(m,1H), 3.0(m,2H), 4.5(m,2H), 6.0(s,1H), 7.1(2xs,2H), 16.0(bs,1H);
3-cyclopropyl-1-(8-methoxy-1,4-benzoxathian-5-yl)propan-1,3-dione;
1-(7-bromo-1,3-benzoxathiolan-4-yl)-3-cyclopropyl-propan-1,3-dione, NMR 0.9(m,2H), 1.2(m,2H), 1.7(m,1H), 5.7(s,2H), 6.25(s,1H), 7.2(s,2H);
3-cyclopropyl-1-(9-fluoro-1,5-benzoxathiepan-6-yl)-propan-1,3-dione, NMR 1.0(m,2H), 1.2(m,2H), 1.7(m,1H), 2.3(m,2H), 3.0(m,2H), 4.4(m,2H), 5.9(s,1H), 6.9(m,1H), 7.1(m,1H), 16.0(bs,1H);
1-(5-chloro-1,4-benzoxathian-8-yl)-3-cyclopropyl-propan-1,3-dione, NMR 0.9(m,2H), 1.2(m,2H), 1.8(m,1H), 3.2(m,2H), 4.5(m,2H), 6.4(s,1H), 7.0(d,1H), 7.5(d,1H), 16.2(bs,1H);
3-cyclopropyl-1-(5-methylthio-1,4-benzoxathian-8-yl)-propan-1,3-dione, NMR 0.9(m,2H), 1.0(m,2H), 1.7(m,1H), 2.4(s,3H), 3.1(m,2H), 4.4(m,2H), 6.4(s,1H), 7.1(d,2H), 16.2(bs,1H);
3-cyclopropyl-1-(8-fluoro-1,4-benzoxathian-5-yl)propan-1,3-dione, NMR 1.0(m,2H), 1.2(m,2H), 1.7(m,1H), 3.0(m,2H), 4.5(m,2H), 6.0(s,1H), 6.9(m,1H), 7.2(m,1H), 16.0(bs,1H);
3-cyclopropyl-1-(8-methylthio-1,4-benzoxathian-5-yl)propan-1,3-dione, NMR 0.9(m,2H), 1.2(m,2H), 2.4(s,3H), 2.6(m,1H), 3.0(m,2H), 4.5(m,2H), 6.1(s,1H), 6.8(m,1H), 7.1-7.3(m,1H), 16.0(bs,1H);
1-[4-bromo-3-(2-chloroethoxy)-2-methylthiophenyl)-3-cyclopropyl-propan-1,3-dione, NMR 1.0(m,2H), 1.2(m,2H), 1.7(m,1H), 2.05(s,3H), 3.9(t,2H), 4.4(t,2H), 5.95(s,1H), 7.1(d,1H), 7.55(d,1H);
3-cyclopropyl-1-(8-methyl-1,4-benzoxathian-5-yl)propan-1,3-dione, NMR 0.9(m,2H), 1.1(m,2H), 1.6(m,1H), 2.1(s,3H), 2.9(m,2H), 4.4(m,2H), 6.0(s,1H), 6.8(d,1H), 7.0(d,1H), 16(bs,1H); and
1-[4-bromo-3-(2-chloro-1-propyloxy)-2-methylthiophenyl]-3-cyclopropyl-propan-1,3-dione, mp66-69°C.

### Reference Example 5

A mixture of 1-(5-chloro-1,4-benzoxathian-8-yl)-3-cyclopropylpropan-1,3-dione (1.0g) and N,N-dimethylformamide-dimethylacetal (0.7 ml) in toluene was heated at 90°C for 2 days. The mixture was then evaporated to give 1-(5-chloro-1,4-benzoxathian-8-yl)-3-cyclopropyl-2-dimethylaminomethylene-propan-1,3-dione.

By proceeding in a similar manner the following compounds were also prepared:
3-cyclopropyl-2-dimethylaminomethylene-1-(8-methoxy-1,4-benzoxathian-5-yl)propan-1,3-dione;
1-(7-bromo-1,3-benzoxathiolan-4-yl)-3-cyclopropyl-2-dimethylaminomethylene-propan-1,3-dione;
3-cyclopropyl-2-dimethylaminomethylene-1-(9-fluoro-1,5-benzoxathiepan-6-yl)-propan-1,3-dione; and
3-cyclopropyl-2-dimethylaminomethylene-1-(5-methylthio-1,4-benzoxathian-8-yl)-propan-1,3-dione.

### Reference Example 6

A mixture of lithium hydroxide (0.33g) and ethyl 3-(1-chloro-2-propyloxy)-4-bromo-2-(methylthio)benzoate (1.94g) in a mixture of water and methanol was stirred at 20°C for 2 days. The mixture was evaporated, diluted with water, filtered, the filtrate acidified (hydrochloric acid) and extracted (ethyl acetate). The organic phase was dried (magnesium sulphate) and evaporated to give 3-(1-chloro-2-propyloxy)-4-bromo-2-(methylthio)benzoic acid (0.88g), NMR 1.5(d, 1H), 2.5(s,3H), 3.75(m,2H), 4.9(m,1H), 7.6(s,2H).

By proceeding in a similar manner the following compounds were also prepared:
5-methylthio-1,4-benzoxathian-8-carboxylic acid, NMR 2.5(s,3H), 3.2(m,2H), 4.4(m,2H), 6.9(d,1H), 7.3(d,1H), 12.5(bs,1H);
8-fluoro-1,4-benzoxathian-5-carboxylic acid, NMR 3.1(m,2H), 4.4(m,2H), 7.0(m,1H), 7.5(m,1H), 13.1(s,1H);
8-methylthio-1,4-benzoxathian-5-carboxylic acid, NMR 2.4(s,3H), 3.0(m,2H), 4.4(m,2H), 6.9(d,1H), 7.6(d,1H);
4-bromo-3-(2-chloroethoxy)-2-(methylthio)benzoic acid, mp93-95°C;
4-bromo-3-(2-chloro-1-propyloxy)-2-(methylthio)benzoic acid, mp95-97°C;
2,4-dibromo-3-(4-methoxybenzyloxy)benzoic acid, mp151 - 154°C;and
7-bromo-1,3-benzoxathiolane-4-carboxylic acid, mp265-269°C.

By proceeding in a similar manner but using potassium hydroxide as base the following compounds were also prepared:
4-bromo-2-(2-fluoroethoxy)-3-methylthiobenzoic acid, NMR 2.4(s,3H), 4.35-4.9(m,4H), 7.5(d,2H), 7.85(d,2H);
8-chloro-1,4-benzoxathian-5-carboxylic acid, NMR 3.0(m,2H), 4.4(m,2H), 7.0(d,1H), 7.5(d,1H);
8-methoxy-1,4-benzoxathian-5-carboxylic acid, NMR 3.0(m,2H), 3.8(s,3H), 4.3(m,2H), 6.8(d,1H), 7.6(d,1H), 12.6(bs,1H);
9-fluoro-1,5-benzoxathiepan-6-carboxylic acid, NMR 2.1(m,2H), 2.9(m,2H), 4.3(m,2H), 6.7(t,1H), 7.4(m,1H);
4-fluoro-3-methoxy-2-methylthiobenzoic acid, mp96-98°C; and
8-methyl-1,4-benzoxathian-5-carboxylic acid, NMR 2.1(s,3H), 3.0(m,2H), 4.4(m,2H), 6.8(d,1H), 7.5(d,1H), 8.0(bs,1H).

### Reference Example 7

A mixture of methyl 4-fluoro-2-(2-fluoroethoxy)-3-methylthiobenzoate (3.0g) and hydrochloric acid (5M) was heated at reflux for 24 hours and cooled. The solid was filtered and recrystallised from toluene to give 4-fluoro-1,4-benzoxathian-8-carboxylic acid (1.8g), mp165-167°C.

### Reference Example 8

A solution of 2-fluoroethanol (6.5g) in dry tetrahydrofuran was added dropwise at 25°C to sodium hydride (60% oil dispersion, 4.0g) stirred in tetrahydrofuran. After 1 hour a solution of methyl 2,4-difluoro-3-(methylthio)benzoate (20g) in tetrahydrofuran was added dropwise at 5°C and stirred for a further 2 hours at 20°C. The mixture was added to ice/water, acidified (hydrochloric acid), extracted(ether) and evaporated to give methyl 4-fluoro-2-(2-fluoroethoxy)-3-methylthiobenzoate (23g), NMR 2.5(s,3H), 3.9(s,3H), 4.35(m,1H), 4.75(m,1H), 4.9(m,1H), 6.95(t,1H), 7.8(q,1H).

By proceeding in a similar manner the following compound was also prepared:
2[4-chlor―2-(2-fluoroethoxy)-3-methylthiophenyl]-4,4-dimethyl-1,3-oxazoline, NMR 1.4(s,6H), 2.5(s,3H), 4.1(s,2H), 4.3(m,2H), 4.7(m,2H), 7.2(d,1H), 7.6(d,1H).

### Reference Example 9

n-Butyllithium (1.5ml of a 2.5M solution in hexanes) was added dropwise under nitrogen at -60°C to a solution of tetrahydrofuran saturated with methanethiol. Further methanethiol was bubbled into the solution and after 0.5 hour a solution of ethyl 2,4-dibromo-3-(1-chloro-2-propyloxy)benzoate (1.43g) in tetrahydrofuran was added. Stirring was maintained at 20°C for 2 days, water added and the mixture extracted (ether), dried (magnesium sulphate), evaporated and chromatographed on silica gel eluting with ethyl acetate/hexane (1:20) to give ethyl 4-bromo-3-(1-chloro-2-propyloxy)-2-(methylthio)benzoate (0.58g), NMR 1.4(t,3H), 1.5(d,3H), 2.4(s,3H), 3.75(m,2H), 4.4(q,2H), 4.9(m,1H), 7.2-7.6(m,2H).

By proceeding in a similar manner the following compound was also prepared:
ethyl 4-bromo-3-(2-chloroethoxy)-2-(methylthio)benzoate, NMR 1.3(t,3H), 2.4(s,3H), 3.8(t,3H), 4.25(t+q,4H), 7.1(d,1H), 7.5(d, 1H)

### Reference Example 10

A mixture of ethyl 4-chloro-3-(2-chloroethoxy)-2-(methylthio)benzoate (8.5g) in N,N-dimethylformamide was stirred at 120°C for 28 hours and evaporated. Water and ether were added and the ether extract washed (water), dried (magnesium sulphate), evaporated and purified by chromatography on silica gel eluting with ethyl acetate/hexane (1:9) to give ethyl 8-chloro-1,4-benzoxathian-5-carboxylate (3.6g), NMR 1.4(t,3H), 3.9(t,2H), 4.3(t,2H), 4.4(q,2H), 7.3(d,1H), 7.5(d,1H).

By proceeding in a similar manner the following compounds were also obtained:
ethyl 8-methoxy-1,4-benzoxathian-5-carboxylate, NMR 1.4(t,3H), 3.1(m,2H), 3.9(s,3H), 4.3(q,2H), 4.5(m,2H), 6.7(d,1H), 7.7(d,1H);
methyl 9-fluoro-1,5-benzoxathiepan-6-carboxylate, NMR 2.3(m,2H), 3.1(m,2H), 3.9(s,3H), 4.5(m,2H), 6.9(t,1H), 7.5(m,1H);
ethyl 8-fluoro-1,4-benzoxathian-5-carboxylate, NMR 1.4(t,3H), 3.1(m,2H), 4.3(q,2H), 4.5(m,2H), 6.8(m,1H), 7.6(m,1H); and methyl 8-methyl-1,4-benzoxathian-5-carboxylate, NMR 2.2(s,3H), 3.0(m,2H), 3.9(s,3H), 4.4(m,2H), 6.9(d,1H), 7.5(d,1H).

### Reference Example 11

Sodium thiomethoxide (2.5g) was added to a solution of ethyl 2,4-dichloro-3-(2-chloroethoxy)benzoate (9.7g) in tetrahydrofuran and stirred overnight at 20°C. The mixture was added to water, acidified (hydrochloric acid), extracted (ethyl acetate) and evaporated to give ethyl 4-chloro-3-(2-chloroethoxy)-2-methylthiobenzoate (8.5g), NMR 1.4(t,3H), 2.5(s,3H), 3.9(m,2H), 4.3(m,4H), 7.3(d,1H), 7.4(d,1H).

By proceeding in a similar manner the following compounds were also prepared:
ethyl 4-fluoro-3-methoxy-2-methylthiobenzoate, NMR 1.4(t,3H), 2.4(s,3H), 4.0(d,3H), 4.4(q,2H), 7.0(m,1H), 7.3(m,1H);
ethyl 5-methylthio-1,4-benzoxathian-8-carboxylate, NMR 1.3(t,3H), 2.4(s,3H), 3.1(m,2H), 4.2(q,2H), 4.4(m,2H), 6.7(d,1H), 7.4(d,1H);
ethyl 8-methylthio-1,4-benzoxathian-5-carboxylate, NMR 1.3(t,3H), 2.4(s,3H), 3.1(m,2H), 4.3(q,2H), 4.5(m,2H), 6.8(d,1H), 7.6(d,1H); and
ethyl 3-(2-chloroethoxy)-4-fluoro-2-methylthiobenzoate, NMR 1.4(t,3H), 2.5(s,3H), 3.8(m,2H), 4.3-4.4(m,4H), 7.0(m,1H), 7.3(m,1H).

By proceeding in a similar manner but employing lithium thiomethoxide the following compound was also obtained:
ethyl 3-(2-chloroethoxy)-4-methoxy-2-methylthiobenzoate;

By proceeding in a similar manner but employing sodium thiomethoxide and acetone as solvent the following compound was also obtained:
methyl 3-(2-chloroethoxy)-4-methyl-2-(methylthio)benzoate (from methyl 3-(2-chloroethoxy)-4-methyl-2-nitrobenzoate).

### Reference Example 12

A mixture of ethyl 2-bromo-3-hydroxy-4-methoxybenzoate (2.83g), 1-bromo-2-chloroethane (1.1ml) and potassium carbonate was stirred at reflux overnight in acetonitrile, and then evaporated. Water and ethyl acetate were added and the organic phase washed (water), dried (magnesium sulphate) and evaporated to give ethyl 2-bromo-3-(2-chloroethoxy)-4-methoxybenzoate (2.9g), NMR 1.4(t,3H), 3.8(m,2H), 3.9(s,3H), 4.2(m,2H), 4.3(q,2H), 6.8(d,1H), 7.6(d,1H).

By proceeding in a similar manner the following compounds were also obtained:
ethyl 2,4-dibromo-3-(2-chloroethoxy)benzoate, NMR 1.4(t,3H), 3.9(t,2H), 4.25(t,2H), 4.35(q,2H), 7.3(d,1H), 7.6(d,1H);
methyl 3-(3-chloro-1-propyloxy)-4-fluoro-2-methylthiobenzoate, NMR 2.2(m,2H), 2.4(s,3H), 3.8(t,2H), 3.9(s,3H), 4.3(t,2H), 7.0(m,1H), 7.3(m,1H);
ethyl 2,4-dibromo-3-(1-chloro-2-propyloxy)benzoate, NMR 1.4(t,3H), 1.5(d,3H), 3.7-3.8(m,2H), 4.4(q,2H), 4.8(m,1H), 7.3(d,1H), 7.6(d,1H);
ethyl 3-(2-chloroethoxy)-2,4-difluorobenzoate, NMR 1.4(t,3H), 3.8(m,2H), 4.3-4.4(m,4H), 6.9(m,1H), 7.7(m,1H);
methyl 3-(2-chloroethoxy)-4-methyl-2-nitrobenzoate, NMR 2.4(s,3H), 3.8(m,2H), 3.9(s,3H), 4.2(m,2H), 7.4(d,1H), 7.7(d,1H);
ethyl 2,4-dibromo-3-(4-methoxybenzyloxy)benzoate; and
ethyl 2,4-dichloro-3-(2-chloroethoxy)benzoate, NMR 1.4(t,3H), 3.9(m,2H), 4.2(m,2H), 4.4(q,2H), 7.3(d,1H), 7.5(d,1H).

By proceeding in a similar manner but using 2-chloropropan-1-ol tosylate and N,N-dimethylformamide as solvent and performing the reaction at 80°C, the following compound was also obtained:
methyl 4-bromo-3-(2-chloro-1-propyloxy)-2-(methylthio)benzoate.

### Reference Example 13

A solution of bromine (12.25g) in dichloromethane was added dropwise at -70°C to a stirred solution of t-butylamine (11.2g) in toluene. A solution of ethyl 3-hydroxy-4-methoxybenzoate (15g) in dichloromethane was added dropwise and the mixture allowed to warm to 20°C whilst stirring overnight. The solid was filtered, dissolved in hydrochloric acid (2M) and extracted (dichloromethane). The organic extract was dried (magnesium sulphate) and evaporated to give ethyl 2-bromo―3-hydroxy-4-methoxybenzoate (12.45g), NMR 1.4(t,3H), 3.9(s,3H), 4.4(q,2H), 6.1(s,1H), 6.8(d,1H), 7.5(d,1H).

### Reference Example 14

By proceeding according to reference example 9 the following compound was obtained in which both thiomethylation and subsequent cyclisation had occurred:
ethyl 7-bromo-1,3-benzoxathiolane-4-carboxylate, mp75-77°C (starting from ethyl 2,4-dibromo-3(2-chloroethoxy)benzoate).

### Reference Example 15

Concentrated sulphuric acid (3ml) was added dropwise to a stirred solution of 4-fluoro-3-hydroxy-2-methylthiobenzoic acid (12.6g) in methanol. The mixture was heated at reflux for 18 hours, evaporated, diluted (water), sodium hydroxide solution (2M) added to pH11 and extracted (ether). The aqueous phase was acidified (hydrochloric acid), extracted (dichloromethane), washed (sodium bicarbonate solution), dried (magnesium sulphate) and evaporated to give methyl 4-fluoro-3-hydroxy-2-methylthiobenzoate (8.2g), NMR 2.4(s,3H), 3.9(s,3H), 7.1(t,1H), 7.3(s,1H), 7.4(m,2H).

By proceeding in a similar manner the following compounds were also obtained:
methyl 3-hydroxy-4-methyl-2-nitrobenzoate, NMR 2.3(s,3H), 3.9(s,3H), 7.0(d,1H), 7.4(d,1H), 10.4(s,1H);
ethyl 2,4-difluoro-3-hydroxybenzoate, NMR 1.3(t,3H), 4.3(q,2H), 6.2(bs,1H), 6.8(m,1H), 7.3(m,1H); and
methyl 4-bromo-3-hydroxy-2-(methylthio)benzoate.

### Reference Example 16

Boron tribromide (28.1 ml) was added dropwise during 1 hour to a stirred mixture of 4-fluoro-3-methoxy-2-(methylthio)benzoic acid (16.08g) in dichloromethane maintained at -70°C under nitrogen. After 1 hour the mixture was warmed at 20°C for 2 hours and hydrochloric acid (2M) added dropwise keeping below 30°C. Water and ethyl acetate were added and the organic phase dried (magnesium sulphate) and evaporated to give 4-fluoro-3-hydroxy-2-(methylthio)benzoic acid, NMR 2.4(s,3H), 7.2(t,1H), 7.6(m,1H), 8.8(bs,1H).

### Reference Example 17

A mixture of 4,4-dimethyl-2-[4-chloro―2-(2-fluoroethoxy)-3-methylthiophenyl]-1,3-oxazoline (9.5g) and hydrochloric acid (5M) was stirred and heated at reflux for 20 hours, cooled and filtered. The solid was washed with water, hexane and ether and dried to give 5-chloro-1,4-benzoxathian-8-carboxylic acid (5.5g), mp160.5-162°C.

### Reference Example 18

Thionyl chloride (36.7g) was added dropwise to a stirred suspension of N-(2-hydroxy-2-methyl-2-propyl) 4-chloro-2-fluoro-3-(methylthio)benzamide (26g) in dry ether maintained at -10°C under nitrogen. The mixture was stirred at 0°C for 6 hours, ice/water added and the aqueous phase brought to pH6 with sodium hydroxide solution (2M) and extracted (dichloromethane and ethyl acetate). The combined organic phases were dried (magnesium sulphate), evaporated and chromatographed on silica gel eluting with cyclohexane/ethyl acetate (98:2) to give 2-[4-chloro―2-fluoro-3-methylthiophenyl]-4,4-dimethyl-1,3-oxazoline (8.7g), NMR 1.4(s,6H), 2.5(s,3H), 4.1(s,2H), 7.25(m,1H), 7.7(m,1H).

### Reference Example 19

A solution of 4-chloro-2-fluoro-3-(methylthio)benzoyl chloride (21.7g) in dry dichloromethane was added dropwise to a solution of 2-amino-2-methylpropan-1-ol (16.2g) stirred in dichloromethane at -20°C under nitrogen. The mixture was allowed to warm to 20°C, stirred for 2 hours and filtered. The filtrate was evaporated to give N-(1-hydroxy-2-methyl-2-propyl)4-chloro-2-fluoro-3-(methylthio)benzamide (26.0g), mp78-80°C.

### Reference Example 20

Oxalyl chloride (3.6ml) followed by N,N-dimethylformamide (2 drops) were added to a stirred suspension of 5-chloro-1,4-benzoxathian-8-carboxylic acid (3.87g) in dichloromethane at 20°C. After 0.5 hour the mixture was heated at reflux for 2 hours, evaporated and a solution of the residue in dichloromethane added dropwise to a solution of triethylamine (3ml) in ethanol at 0°C. The mixture was stirred overnight at 20°C, evaporated and partitioned between ether and water. The ethereal phase was washed (brine), dried (magnesium sulphate)and evaporated to give ethyl 5-chloro-1,4-benzoxathianane-8-carboxylate (3.6g), NMR 1.3(t,3H), 3.2(m,2H), 4.3(q,2H), 4.4(m,2H), 7.0(d,1H), 7.4(d,1H).

### Reference Example 21

A solution of trifluoroacetic acid in dichloromethane was added dropwise to a stirred mixture of 4-bromo-3-(4-methoxybenzyloxy)-2-(methylthio)benzoic acid (31.2g) in dichloromethane at 20°C under nitrogen. After 3 hours the solvent was evaporated and water and dichloromethane added. The organic phase was washed (water), dried (magnesium sulphate), evaporated and the residue recrystallised to give 4-bromo-3-hydroxy-2-(methylthio)benzoic acid (16.1g), mp145-147°C.

### Reference Example 22

2,4-Dibromo-3-(4-methoxybenzyloxy)benzoic acid (49.5g) was added slowly at 30°C to a stirred mixture of sodium hydride (60%, 10.5g in hexane), in dry dioxan under nitrogen. After 20 minutes sodium thiomethoxide (18g) and copper(I)bromide (5.2g) were added and the mixture heated at reflux for 6 hours. An additional amount of sodium hydride (0.48g), sodium thiomethoxide (0.8g) and copper(I)bromide (0.5g) was added and the mixture heated at reflux for 1.5 hours. The cooled mixture was acidified (2M hydrochloric acid), filtered (hyflo), extracted (ether) and the extract washed (water), dried (magnesium sulphate) and evaporated to give 4-bromo-3-(4-methoxybenzyloxy)-2-(methylthio)benzoic acid (19.3g), mp124-127°C.

According to a feature of the present invention, there is provided a method for controlling the growth of weeds (i.e. undesired vegetation) at a locus which comprises applying to the locus a herbicidally effective amount of at least one isoxazole or 2-cyano-1,3-dione derivative of formula (I) or an agriculturally acceptable salt thereof. For this purpose, the isoxazole or 2-cyano-1,3-dione derivatives are normally used in the form of herbicidal compositions (i.e. in association with compatible diluents or carriers and/or surface active agents suitable for use in herbicidal compositions), for example as hereinafter described.

The compounds of formula (I) show herbicidal activity against dicotyledonous (i.e. broad-leafed) and monocotyledonous (i.e. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil.

For example, the compounds of formula (I) may be used to control the growth of:
broad-leafed weeds, for example, Abutilon theophrasti, Acanthospermum hispidum, Amaranthus retroflexus, Bidens pilosa, Chenopodium album, Euphorbia heterophylla, Galium aparine, Ipomoea spp. e.g. Ipomoea purpurea and Ipomoea grandiflora, Sesbania exaltata, Sida praziovii, Sinapis arvensis, Solanum nigrum and Xanthium strumarium, and
grass weeds, for example Alopecurus myosuroides, Avena fatua, Brachiaria decumbens, Cenchrus echinatus, Digitaria sanguinalis, Echinochloa crus-galli, Eleucine indica, Sorghum bicolor, Eleusine indica and Setaria spp, e.g. Setaria faberii or Setaria viridis, and
sedges, for example, Cyperus esculentus.

The amounts of compounds of formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates from 0.01kg to 2kg, preferably 0.01kg to 1kg, of active material per hectare give good results. However, it is to be understood that higher or lower application rates may be used, depending upon the particular problem of weed control encountered.

The compounds of formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soybean, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates from 0.01 kg to 1.0kg, and preferably from 0.01 kg to 0.4kg, of active material per hectare are particularly suitable.

The compounds of formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non- directional fashion (e.g. by directional or non-directional spraying) to the weeds or to the soil in which they are expected to appear, before or after planting of the trees or plantations at application rates from 0.05kg to 1.0kg, preferably from 0.1 kg to 0.4kg, of active material per hectare.

The compounds of formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates from 0.05kg to 1.0kg, preferably from 0.1kg to 0.75kg, and more preferably from 0.2kg to 0.5kg of active material per hectare are particularly suitable for this purpose.

When used to control the growth of weeds by pre-emergence application, the compounds of formula (I) may be incorporated into the soil in which the weeds are expected to emerge. It will be appreciated that when the compounds of formula (I) are used to control the growth of weeds by post-emergence application, i.e. by application to the aerial or exposed portions of emerged weeds, the compounds of formula (I) will also normally come into contact with the soil and may also then exercise a pre-emergence control on later-germinating weeds in the soil.

Where especially prolonged weed control is required, the application of the compounds of formula (I) may be repeated if required.

According to a further feature of the present invention, there are provided compositions suitable for herbicidal use comprising one or more of the isoxazole or 2-cyano-1,3-dione derivatives of formula (I), in association with, and preferably homogeneously dispersed in, one or more compatible agriculturally- acceptable diluents or carriers and/or surface active agents [i.e. diluents or carriers and/or surface active agents ofthe type generally accepted in the art as being suitable for use in herbicidal compositions and which are compatible with compounds of formula (I)]. The term "homogeneously dispersed" is used to include compositions in which the compounds of formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, tale, calcined magnesia, kieselguhr, tricalcium phosphate, powdered cork, absorbent carbon black and clays such as kaolin and bentonite. The solid compositions (which may take the form of dusts, granules or wettable powders) are preferably prepared by grinding the compounds of formula (I) with solid diluents or by impregnating the solid diluents or carriers with solutions of the compounds of formula (I) in volatile solvents, evaporating the solvents and, if necessary, grinding the products so as to obtain powders. Granular formulations may be prepared by absorbing the compounds of formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, spreading agents, stabilisers, sequestering agents, anti-caking agents, colouring agents and corrosion inhibitors. These adjuvants may also serve as carriers or diluents.

Herbicidal compositions according to the present invention may also comprise the compounds of formula I in association with, and preferably homogeneously dispersed in, one or more other pesticidally active compounds and, if desired, one or more compatible pesticidally acceptable diluents or carriers, surface-active agents and conventional adjuvants as hereinbefore described.

Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2-dimethyl-3,5-diphenyl-pyrazolium salts], flampropmethyl [methyl N-2-(N- benzoyl-3-chloro-4-fluoroanilino)-propionate], fluometuron [N'-(3-trifluoro-methylphenyl)-N,N-dimethylurea], isoproturon [N'-(4-isopropylphenyl)-N,N-dimethylurea], insecticides, e.g. synthetic pyrethroids, e.g permethrin and cypermethrin, and fungicides, e.g. carbamates, e.g. methyl N-(1-butyl-carbamoylbenzimidazol-2-yl)carbamate, and triazoles e.g. 1-(4-chloro-phenoxy)-3,3-dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilised in the form of conventional derivatives, for example alkali metal and amine salts and esters.

The following Examples illustrate herbicidal compositions according to the present invention. The following trade marks appear in the Examples: Synperonic, Solvesso, Arylan, Arkopon, Sopropon, Tixosil, Soprophor, Attagel, Rhodorsil.

### Example C1:

An emulsifiable concentrate is formed from:

| | |
|---|---|
| Active ingredient (Compound 1) | 20% w/v |
| N-Methylpyrrolidinone (NMP) | 25% w/v |
| Calcium dodecylbenzenesulphonate 70% (CaDDBS) (Arylan CA) | 4% w/v |
| Nonylphenol ethylene oxide propylene oxide condensate (NPEOPO) (Synperonic NPE 1800) | 6% w/v |
| Aromatic solvent (Solvesso) | to 100 volumes |

by stirring NMP, active ingredient (Compound 1), CaDDBS, NPEOPO and Aromatic solvent until a clear solution is formed, and adjusting to volume with Aromatic solvent.

### Example C2

A wettable powder is formed from:

| | |
|---|---|
| Active ingredient (Compound 1) | 50% w/w |
| Sodium dodecylbenzenesulphonate (Arylan SX85) | 3% w/w |
| Sodium methyl oleoyl taurate (Arkopon T) | 5% w/w |
| Sodium polycarboxylate (Sopropon T36) | 1% w/w |
| Microfine silicon dioxide (Tixosil 38) | 3% w/w |
| China clay | 38% w/w |

by blending the above ingredients together and grinding the mixture in an air jet mill.

### Example C3

A suspension concentrate is formed from:

| | |
|---|---|
| Active ingredient (Compound 1) | 50% w/v |
| Antifreeze (Propylene glycol) | 5% w/v |
| Ethoxylated tristyrylphenol phosphate (Soprophor FL) | 0.5% w/v |
| Nonyl phenol 9 mole ethoxylate (Ethylan BCP) | 0.5% w/v |
| Sodium polycarboxylate (Sopropon T36) | 0.2% w/v |
| Attaclay (Attagel) | 1.5% w/v |
| Antifoam (Rhodorsil AF426R) | 0.003% w/v |
| Water | to 100 volumes |

by stirring the above ingredients together and milling in a bead mill.

### Example C4

A water dispersible granule is formed from:

| | |
|---|---|
| Active ingredient (Compound 1) | 50% w/w |
| Sodium dodecylbenzenesulphonate (Arylan SX 85) | 3% w/w |
| Sodium methyl oleoyl taurate (Arkopon T) | 5% w/w |
| Sodium polycarboxylate (Sopropon T36) | 1% w/w |
| Binder (Sodium lignosulphonate) | 8% w/w |
| China clay | 30% w/w |
| Microfine silicon dioxide (Tixosil 38) | 3% w/w |

by blending the above ingredients together, grinding the mixture in an airjet mill and granulating by addition of water in a suitable granulation plant (e.g. Fluid bed drier) and drying. Optionally the active ingredient may be ground either on its own or admixed with some or all of the other ingredients.

The compounds of the invention have been used in herbicidal applications according to the following procedures.

### METHOD OF USE OF HERBICIDAL COMPOUNDS:

### TEST METHOD

### a) General

Appropriate quantities of the compounds used to treat the plants were dissolved in acetone to give solutions equivalent to application rates of up to 250g test compound per hectare (g/ha). These solutions were applied from a standard laboratory herbicide sprayer delivering the equivalent of 290 litres of spray fluid per hectare.

### b) Weed control : Pre-emergence

The seeds were sown in 70 mm square, 75 mm deep plastic pots in non-sterile soil. The quantities of seed per pot were as follows:-

| Weed species | Approx number of seeds/pot |
|---|---|
| | |

| 1) Broad-leafed weeds | |
|---|---|
| Abutilon theophrasti | 10 |
| Amaranthus retroflexus | 20 |
| Galium aparine | 10 |
| Ipomoea purpurea | 10 |
| Xanthium strumarium | 2 |

| 2) Grass weeds | |
|---|---|
| Alopecurus myosuroides | 15 |
| Avena fatua | 10 |
| Echinochloa crus-galli | 15 |
| Setaria viridis | 20 |

| 3) Sedges | |
|---|---|
| Cyperus esculentus | 3 |

| Crop | |
|---|---|
| | |

| 1) Broad-leafed | |
|---|---|
| Cotton | 3 |
| Soybean | 3 |

| 2) Grass | |
|---|---|
| Maize | 2 |
| Rice | 6 |
| Wheat | 6 |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead . Visual assessment of crop damage was made 20-24 days after spraying. The results were expressed as the percentage reduction in growth or damage to the crop or weeds, in comparison with the plants in the control pots.

### c) Weed control : Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-

| 1) Broad leafed weeds | | |
|---|---|---|
| Weed species | No. of plants/pot | Growth stage |
| Abutilon theophrasti | 3 | 1-2 leaves |
| Amaranthus retroflexus | 4 | 1-2 leaves |
| Galium aparine | 3 | 1^{st} whorl |
| Ipomoea purpurea | 3 | 1-2 leaves |
| Xanthium strumarium | 1 | 2-3 leaves |

| 2) Grass weeds | | |
|---|---|---|
| Weed species | No. of plants/pot | Growth stage |
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

| 3) Sedges | | |
|---|---|---|
| Weed species | No. of plants/pot | Growth stage |
| Cyperus esculentus | 3 | 3 leaves. |

| 1) Broad leafed | | |
|---|---|---|
| Crops | No. of plants/pot | Growth stage |
| Cotton | 2 | 1 leaf |
| Soybean | 2 | 2 leaves. |

| 2) Grass | | |
|---|---|---|
| Crops | No. of plants/pot | Growth stage |
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

When applied pre- or post-emergence in the above Test Method at 250g/ha or less compounds 1-27, 29-32 and 34-38 of the invention gave at least 90% reduction in growth of one or more of the weed species; at levels of application toxic to the weeds these compounds were selective in at least one of the crop species.

## Claims

1. A 4-aroylisoxazole derivative of formula (Ia) or a 2-cyano-1,3-dione derivative of formula (Ib): 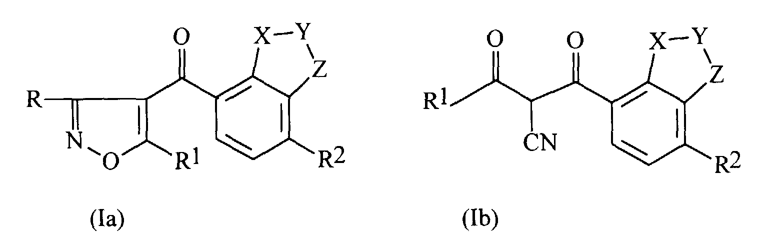 wherein:
R represents hydrogen or -CO₂R³;
R¹ represents:-
cyclopropyl or 1-methylcyclopropyl;
R² represents:-
halogen, lower alkyl, lower haloalkyl, lower alkoxy, lower haloalkoxy, -S(O)ₘR⁴ or SF₅;
m represents zero, one or two;
X, Y and Z together with the two attached carbon atoms form a fused 5- to 7- membered heterocyclic ring which is aromatic or partially or fully saturated containing from one to three heteroatoms which may be the same or different selected from nitrogen, oxygen and sulphur with at least one atom being a sulphur atom (the sulphur atoms being optionally in the SO or SO₂ oxidation states), and the ring carbon atoms being optionally substituted by one or two groups selected from halogen, lower alkyl, lower haloalkyl, lower alkoxy and lower haloalkoxy, one of the ring carbon atoms optionally forming part of a carbonyl group or an oxime or lower alkoxyimine derivative thereof; and optionally two of the adjacent carbon atoms which when present form part ofthe X-Y-Z group in the heterocyclic ring may themselves form part of a second fused pyrazole or isoxazole ring optionally substituted by lower alkyl, lower haloalkyl, halogen or -CO₂R⁵;
R³ represents lower alkyl or lower haloalkyl;
R⁴ represents lower alkyl, lower haloalkyl, lower alkenyl, lower haloalkenyl, lower alkynyl, lower haloalkynyl or cycloalkyl containing from three to six carbon atoms;
R⁵ represents lower alkyl; or an agriculturally acceptable salt or metal complex thereof.

2. A compound according to claim 1 which is of formula (Ia).

3. A compound according to claim 1 or 2 in which R represents hydrogen.

4. A compound according to claim 1, 2 or 3 in which R¹ represents cyclopropyl.

5. A compound according to any one of the preceding claims in which R² is selected from fluorine, chlorine, bromine, methyl, methoxy and -S(O)ₘR⁴ where R is methyl.

6. A compound according to any one of the preceding claims in which the -X-Y-Z- group together with the phenyl ring to which it is fused forms a ring system which is selected from thiochroman, 2H-thiochromene, 4H-thiochromene, isothiochroman, isothiochromene, 1,3-benzodithiole, 1,3-benzoxathiole, 1,4-benzodithiin, 1,4-benzodithian, 1,4-benzoxathiin, 1,4-benzoxathian, 1,3-benzoxathian, 3,1 - benzoxathian, 1,3-benzodithian, benzo[b]thiophene, 2,3-dihydrobenzo[b]thiophene, 3-oxo-2,3-dihydro-benz[d]isothiazole, 1,5-benzoxathiepan, benzothiopyrano[4,3-c]pyrazole, benzothiopyrano[4,3-c]isoxazole and benzothiopyrano[3,4-d]isoxazole.

7. A compound of formula (Ia) according to claim 1 in which:
R represents hydrogen or -CO₂R³;
R¹ represents cyclopropyl;
R² represents:-
halogen; a straight- or branched- chain alkyl group containing up to four carbon atoms optionally substituted by one or more halogen atoms; -S(O)ₘR⁴ or a straight- or branched- chain alkoxy group containing from one to four carbon atoms;
R³ and R⁴ each represent a straight- or branched- chain alkyl group containing from one to four carbon atoms;
X represents S(O)ₙ, O or C(=O);
Y represents (CH₂)ₚ optionally substituted by lower alkyl;
Z represents S(O)_{q}, O, C(=O), C(=NOH), C(=NOCH₃) or CH(OCH₃);
provided that at least one of the groups S(O)ₙ or S(O)_{q} is present as the group X or Z respectively;
n and q independently represent zero, one or two; and
p represents one, two or three; or the -X-Y-Z- group together with the phenyl ring to which it is fused forms a ring system which is selected from benzothiopyrano[4,3-c]pyrazole, benzothiopyrano[4,3-c]isoxazole or benzothiopyrano[3,4-d]isoxazole optionally substituted in the pyrazole or isoxazole ring by lower alkyl or lower alkoxycarbonyl.

8. A compound of formula (Ia) according to claim 1 in which:
R represents hydrogen or -CO₂R³;
R¹ represents cyclopropyl;
R² represents:-
halogen, optionally halogenated methyl, -S(O)ₘCH₃ or OCH₃;
R³ represents methyl or ethyl;
Y represents (CH2)ₚ optionally substituted by lower alkyl;
p represents one, two or three; and
X represents S(O)ₙ and Z represents O; or X represents O and Z represents S(O)_{q}.

9. A herbicidal composition comprising an effective amount of at least one compound of formula (Ia) or (Ib) according to any one of claims 1 to 8 or an agriculturally acceptable salt or metal complex thereof, in association with an agriculturally acceptable diluent or carrier and/or surface active agent.

10. A method for the control of weeds at a locus which comprises applying to said locus an effective amount of at least one compound of formula (Ia) or (Ib) according to any one of claims 1 to 8 or an agriculturally acceptable salt or metal complex thereof or a herbicidal composition according to claim 9.

11. A method according to claim 10 wherein the locus is an area used, or to be used, for the growing of crops and the compound of formula (Ia) or (Ib) or salt or metal complex thereof is applied at an application rate of from 0.01 kg to 1 kg/ha.

12. A process for the preparation of a compound of formula (Ia) or (Ib) as defined in claim 1 which comprises:
(a) where the compound is of formula (Ia) in which R represents hydrogen and R¹, R², X, Y and Z are as defined in claim 1, the reaction of a compound of formula (II): 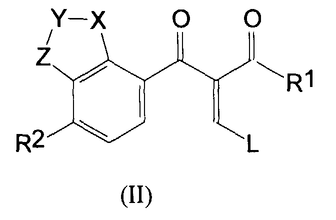 where L is a leaving group and R¹, R², X, Y and Z are as defined in claim 1, with hydroxylamine or a salt of hydroxylamine;
(b) where the compound is of formula (Ia) in which R represents hydrogen and R¹, R², X, Y and Z are as defined in claim 1, the reaction of a compound of formula (III): 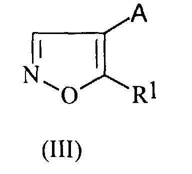 wherein R¹ is as defined in claim 1 and A represents a carboxy group or a reactive derivative thereof or a cyano group, with an appropriate organometallic reagent;
(c) where the compound is of formula (Ia) wherein R represents -CO₂R³ and R¹, R², X, Y and Z are as defined in claim 1, the reaction of a salt of a compound of formula (IV): 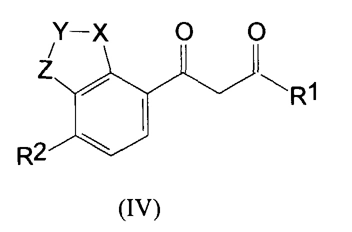 wherein R¹, R², X, Y and Z are as defined in claim 1 with a compound of formula R³O₂CC(E)=NOH wherein R³ is as defined in claim 1 and E represents halogen;
(d) where the compound is of formula (Ia) wherein R, R¹ and R² are as defined in claim 1, Y represents (CH₂)ₚ optionally substituted by lower alkyl, and one of X and Z represents S and the other represents O, the reaction of the corresponding compound of formula (V) or (VI): 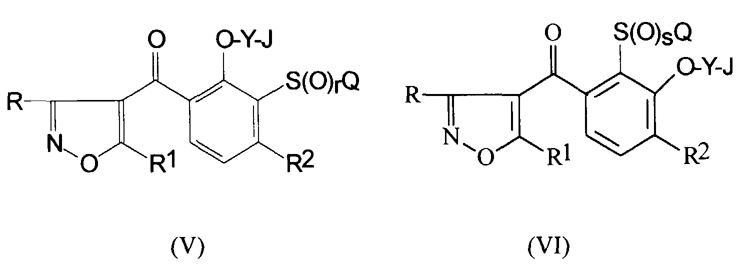 wherein R, R¹ and R² are as defined in claim 1, Y represents (CH₂)ₚ optionally substituted by lower alkyl, Q represents lower alkyl, J represents a leaving group, and r and s represent one, with an anhydride via a thiol intermediate of formula (V) or (VI) in which Q is replaced by hydrogen, followed by cyclisation;
(e) where the compound is of formula (Ia) wherein R, R¹ and R² are as defined in claim 1, Y represents (CH₂)ₚ optionally substituted by lower alkyl, and one of X and Z represents S and the other represents O, the thermal cyclisation of the corresponding compound of formula (V) or (VI) wherein R, R¹ and R² are as defined in claim 1, Q represents lower alkyl, J represents a leaving group, and r and s represent zero;
(f) where the compound is of formula (Ib) in which R¹, R², X, Y and Z are as defined in claim 1, treating the corresponding compound of formula (Ia) in which R represents a hydrogen atom with a base;
(g) where the compound is of formula (Ib) in which R¹, R², X, Y and Z are as defined in claim 1, hydrolysing the corresponding compound of formula (Ia) in which R represents -CO₂R³ and R³ is defined in claim 1;
(h) where the compound is of formula (Ib) in which R¹, R², X, Y and Z are as defined in claim 1, the reaction of an acid chloride of formula (VII): 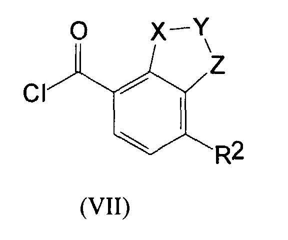 wherein R², X, Y and Z are as defined in claim 1, with a beta-ketonitrile of formula (VIII):
R¹C(=O)CH₂CN (VIII)
wherein R¹ is as defined in claim 1;
(i) where the compound is of formula (Ib) in which R¹, R², X, Y and Z are as defined in claim 1, the reaction of an acid chloride of formula R¹COCl wherein R¹ is as defined in claim 1, with a beta-ketonitrile of formula (IX): 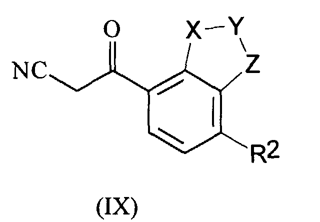 wherein R², X, Y and Z are as defined in claim 1;
(j) where the compound is of formula (Ib) in which R¹, R², X, Y and Z are as defined in claim 1, the reaction of a carboxylic acid chloride of formula (VII) above wherein R², X, Y and Z are as defined in claim 1, with a beta-ketonitrile of formula (VIII) wherein R¹ is as defined in claim 1, via an intermediate of formula (X): 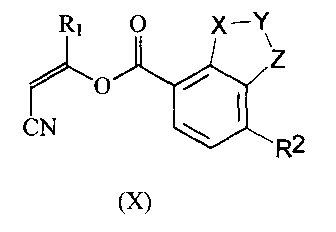 wherein R¹, R², X, Y and Z are as defined in claim 1;
(k) where the compound is of formula (Ib) in which R¹, R², X, Y and Z are as defined in claim 1, the reaction of an acid chloride of formula R¹COCl wherein R¹ is as defined in claim 1, with a betaketonitrile of formula (IX) wherein R², X, Y and Z are as defined in claim 1 via an intermediate of formula (XI): 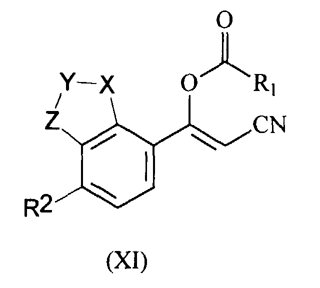 wherein R¹, R², X, Y and Z are as defined in claim 1;
(l) where the compound is of formula (I) in which R, R¹, R², X, Y and Z are as defined in claim 1 wherein one or more of the sulphur atoms is at the sulphoxide or sulphone oxidation state, the oxidation of the sulphur atom of the corresponding compound of formula (I) in which the sulphur atom to be oxidised is at a lower oxidation state; optionally followed by the conversion of the compound of formula (I) thus obtained into an agriculturally acceptable salt or metal complex thereof.

13. A compound of formula (II), (IV), (XIV) and (XVI): 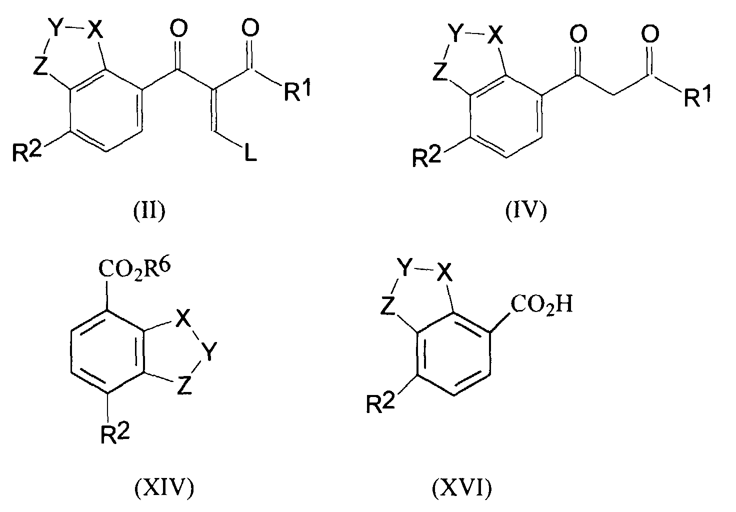 wherein the various substituents are as defined in any one of the previous claims.
